(19) **Europäisches Patentamt / European Patent Office / Office européen des brevets**

(11) **EP 3 807 635 B1**

(12) # FASCICULE DE BREVET EUROPEEN

(45) Date de publication et mention de la délivrance du brevet:
**09.04.2025 Bulletin 2025/15**

(21) Numéro de dépôt: **19730387.8**

(22) Date de dépôt: **18.06.2019**

(51) Classification Internationale des Brevets (IPC):
**G01N 33/50** (2006.01)    **C12Q 1/6883** (2018.01)
**C12Q 1/70** (2006.01)

(52) Classification Coopérative des Brevets (CPC):
**G01N 33/5091; C12Q 1/6883; C12Q 1/701;**
C12Q 2600/156; G01N 2333/025;
G01N 2333/70539; G01N 2800/245; G01N 2800/52;
G01N 2800/60

(86) Numéro de dépôt international:
**PCT/EP2019/066100**

(87) Numéro de publication internationale:
**WO 2019/243372 (26.12.2019 Gazette 2019/52)**

(54) **MÉTHODE DE STRATIFICATION DU RISQUE DE NÉPHROPATHIE À BK-VIRUS APRÈS TRANSPLANTATION RÉNALE**

VERFAHREN ZUR STRATIFIZIERUNG DES RISIKOS EINER BK-VIRUS-NEPHROPATHIE NACH EINER NIERENTRANSPLANTATION

METHOD FOR STRATIFYING THE RISK OF BK VIRUS NEPHROPATHY AFTER A KIDNEY TRANSPLANT

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **18.06.2018 FR 1855342**

(43) Date de publication de la demande:
**21.04.2021 Bulletin 2021/16**

(73) Titulaires:
• **Université Paris-Saclay**
**91190 Gif-sur-Yvette (FR)**
• **Institut National de la Santé et de la Recherche Médicale (INSERM)**
**75013 Paris (FR)**
• **Assistance Publique - Hôpitaux de Paris**
**75012 Paris (FR)**

(72) Inventeurs:
• **TAOUFIK, Yassine**
**75014 Paris (FR)**
• **DURRBACH, Antoine**
**75016 Paris (FR)**
• **DEKEYSER, Manon**
**75013 Paris (FR)**

(74) Mandataire: **Regimbeau**
**20, rue de Chazelles**
**75847 Paris Cedex 17 (FR)**

(56) Documents cités:
**US-A1- 2018 127 838**

• **OSCAR M. PELLO ET AL: "BKV-specific T cells in the treatment of severe refractory haemorrhagic cystitis after HLA-haploidentical haematopoietic cell transplantation", EUROPEAN JOURNAL OF HAEMATOLOGY., vol. 98, no. 6, 1 March 2017 (2017-03-01), DK, pages 632 - 634, XP055606528, ISSN: 0902-4441, DOI: 10.1111/ejh.12848**
• **BYUNG HA CHUNG ET AL: "Clinical usefulness of BK virus plasma quantitative PCR to prevent BK virus associated nephropathy : Clinical usefulness of plasma BK virus real time PCR", TRANSPLANT INTERNATIONAL., vol. 25, no. 6, 18 April 2012 (2012-04-18), GB, pages 687 - 695, XP055552293, ISSN: 0934-0874, DOI: 10.1111/j.1432-2277.2012.01480.x**

- **MANON DEKEYSER ET AL: "Polyomavirus-Specific Cellular Immunity: From BK-Virus-Specific Cellular Immunity to BK-Virus-Associated Nephropathy?", FRONTIERS IN IMMUNOLOGY, vol. 6, 16 June 2015 (2015-06-16), XP055552110, DOI: 10.3389/fimmu.2015.00307**
- **B SULEIMAN ET AL: "HLA Mismatching and cPRA Do Not Affect BK Viremia and Nephropathy Risk in Kidney Transplantation: A Retrospective Analysis - ATC Abstracts", AMERICAN JOURNAL OF TRANSPLANTATION, 4 June 2018 (2018-06-04), XP055553215, Retrieved from the Internet <URL:https://atcmeetingabstracts.com/abstract/hla-mismatching-and-cpra-do-not-affect-bk-viremia-and-nephropathy-risk-in-kidney-transplantation-a-retrospective-analysis/> [retrieved on 20190207]**
- **LANOT ANTOINE ET AL: "Infections à BK virus en transplantation rénale", NEPHROLOGIE & THERAPEUTIQUE, ELSEVIER, NL, vol. 12, no. 2, 28 January 2016 (2016-01-28), pages 76 - 85, XP029448127, ISSN: 1769-7255, DOI: 10.1016/J.NEPHRO.2015.11.003**
- **MANON DEKEYSER: "Anomalies de la mémoire lymphocytaire T antivirale et infections virales en transplantation rénale", THESES.FR, 18 February 2019 (2019-02-18), XP055606774, Retrieved from the Internet <URL:http://www.theses.fr/2019SACLS065#> [retrieved on 20190718]**

Remarques:
> Le document complet y compris le(s) table(s) de référence et le(s) listage(s) de séquence sont téléchargeables sur le site internet de l'OEB

# EP 3 807 635 B1

**Description**

**Résumé de l'invention**

La présente demande décrit et revendique une nouvelle méthode pour identifier et stratifier le risque de développer une néphropathie à BK-virus (ci-après « Nx BK-v ») chez les patients ayant subi une transplantation rénale telle que définie dans les revendications. Cette méthode utilise un index combinant au moins trois biomarqueurs : i) l'intensité de la réponse lymphocytaire T mémoire anti-BK-v (lymphocytes T mémoires spécifiques du BK-v, ci-après « LTm anti-BK-v »), ii) le nombre d'incompatibilités au niveau des allèles HLA de classe I et de classe II entre le donneur et le receveur du greffon, en tenant compte de iii) la charge virale du virus BK-v dans le sang total du patient. La présente méthode permet d'évaluer très précisément le risque de développer une Nx BK-v dans les mois suivant le test, dans le but d'optimiser le traitement immunosuppresseur, pour mieux préserver le rein greffé.

**Description de l'art antérieur**

Les abréviations suivantes ont été utilisées dans la présente demande :

- BK-v *: Polyomavirus* BK humain
- CFSE: *Carboxyfluorescein succinimidyl ester,* marqueur de prolifération lymphocytaire
- HLA : Human Leukocyte Antigens
- JC-v *: Polyomavirus* JC humain
- LTm anti-BK-v : lymphocytes T mémoires spécifiques du BK-v
- Nx BK-v : néphropathie à BK-virus
- Réponse LTm anti-BK-v : réponse lymphocytaire T mémoire spécifique du BK-v

<u>L'insuffisance rénale chronique terminale et ses traitements de suppléance</u>
L'insuffisance rénale chronique terminale représente l'altération complète et définitive de la fonction rénale nécessitant le recours à un traitement de suppléance rénale (par dialyse ou transplantation). En France, pour l'année 2015, la prévalence de l'insuffisance rénale chronique terminale était de 82 295 patients, soit 1 232 pmh.
La transplantation rénale constitue actuellement le traitement le plus efficace de l'insuffisance rénale chronique terminale. Comparée au maintien en dialyse, la transplantation rénale améliore la survie et la qualité de vie des patients, avec un coût financier moindre. Il s'agit de la plus grande activité de transplantation d'organe solide dans le monde.
En 2014, l'observatoire global pour le don d'organe et la transplantation (GODT, organisme émanant de l'Organisation Mondial de la Santé) recensait 119 873 patients transplantés au niveau mondial, dont 79 948 (67%) patients transplantés rénaux. En France, en 2015, le nombre estimé de patients porteurs d'un greffon rénal fonctionnel était de 36 729, soit une prévalence de 552,4 cas par million d'habitants, représentant ainsi 44,6 % des 82 295 patients traités pour insuffisance rénale chronique terminale.
Du fait de la pénurie actuelle d'organe pour satisfaire l'ensemble des besoins en transplantation rénale (1 greffon rénal disponible pour 4,7 personnes en attente avec un délai médian d'attente de 18,5 mois), il est indispensable d'accroitre la durée de vie des greffons rénaux actuellement estimée à 13 ans ([1], [2]). Dans ce but, les patients transplantés sont systématiquement soumis à une immunosuppression thérapeutique. Cette mesure est en effet indispensable pour contrôler la réponse immune allogénique et augmenter la survie du greffon. Cependant, l'immunosuppression thérapeutique peut être associée à des complications, en particulier infectieuses, pouvant être délétères pour le greffon. Ces infections peuvent conduire à la perte du rein transplanté, imposant alors au patient un retour en dialyse (avec toutes les conséquences néfastes en découlant) et entrainant une augmentation du nombre de malades en attente de greffe rénale.

<u>Réactivation des *Polyomavirus* BK et JC en transplantation rénale</u>

Les réactivations virales sont des complications très fréquentes chez les patients immunodéprimés. Notamment, la réactivation de certains *Polyomavirus* est particulièrement préoccupante. Chez l'homme, 13 espèces de *Polyomavirus* sont actuellement connues. Il s'agit de virus ubiquitaires, infectant de nombreuses espèces animales avec une spécificité d'espèce. La séroprévalence de ces infections virales est très élevée au niveau mondial (80 - 90 % à l'âge adulte), indiquant une exposition virale précoce et large. Les deux principaux représentants sont les virus BK (BK-v) et JC (JC-v). La caractéristique commune de ces virus est leur capacité à persister au long cours de manière asymptomatique dans l'organisme, et à devenir pathogènes en situation d'immunosuppression ([3], [4]).
Après transplantation, le BK-v est connu pour infecter les cellules épithéliales du tractus urinaire et le parenchyme rénal du rein transplanté. Chez les transplantés rénaux, la réactivation du virus BK peut mener à une charge virale détectable dans le sang (> 200 copies/mL).

Ainsi, dans le cadre de la transplantation rénale, le BK-v est responsable de néphropathies (ci-après Nx BK-v), d'insuffisance rénale ou de sténoses urétérales ([4]). Dans le cadre des greffes de cellules souches hématopoïétiques, le BK-v est quant à lui responsable de cystites hémorragiques ([4]).

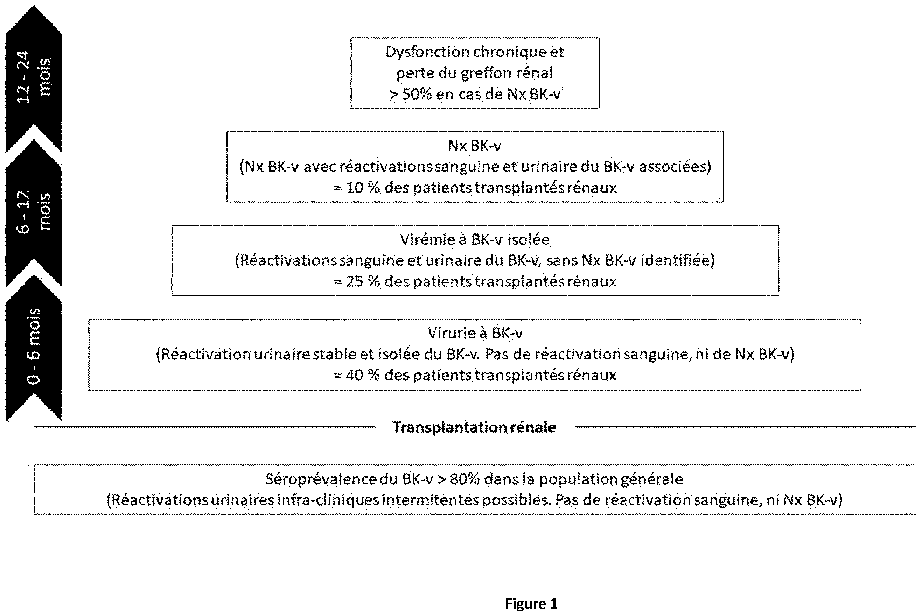

Plusieurs niveaux de réactivations à BK-v sont observés chez les patients ayant subi une transplantation rénale (figure 1).

Les patients peuvent présenter :

- 1- Une « virurie à BK-v » sans virémie ni atteinte rénale détectable. Chez ces patients, le BK-v réplique dans le tractus urinaire. Une virurie est observée chez près de 40% des patients transplantés rénaux, et survient classiquement dans les 3 à 6 premiers mois post-greffe.

- 2- Une « virémie à BK-v isolée » sans atteinte rénale spécifique détectable du greffon rénal. Une telle virémie est observée chez près de 24% des patients transplantés rénaux et survient classiquement dans les 12 premiers mois de la transplantation rénale.

- 3- Une Nx BK-v. Chez ces patients, il est observé non seulement une virémie et une virurie mais aussi une réplication virale au niveau du greffon rénal, responsable d'une atteinte rénale spécifique. Le diagnostic est actuellement histologique et met en évidence des inclusions virales nucléaires (antigènes T de SV40 positif), des dystrophies nucléaires, une infiltration cellulaire polymorphe et une fibrose rénale. Cette grave complication est diagnostiquée chez près de 10% des patients transplantés rénaux et survient classiquement dans les 24 premiers mois de la transplantation rénale ([5]-[6]). L'évolution d'une Nx BK-v dépend de l'étendue des lésions du parenchyme rénal, elle-même liée à l'intensité de la charge virale (cf. tableau 8 de Hirsch et al, 2014 [7]). L'évolution est favorable chez les patients dont le système immunitaire réussit à contrôler la réplication intra-parenchymateuse du BK-v. En revanche, la persistance d'une réplication virale intra-parenchymateuse intense et prolongée du BK-v mène à des lésions irréversibles du parenchyme rénal, entraînant une dysfonction chronique, une insuffisance rénale et aboutissant à une perte de greffon dans plus de 50% des cas.

Le tableau 8 de Hirsch et al, Clinical Microbiology and Infection 2014 [7] montre les différents stades histologiques de la Nx BK-v, ainsi que le pronostic rénal associé à l'atteinte histologique.

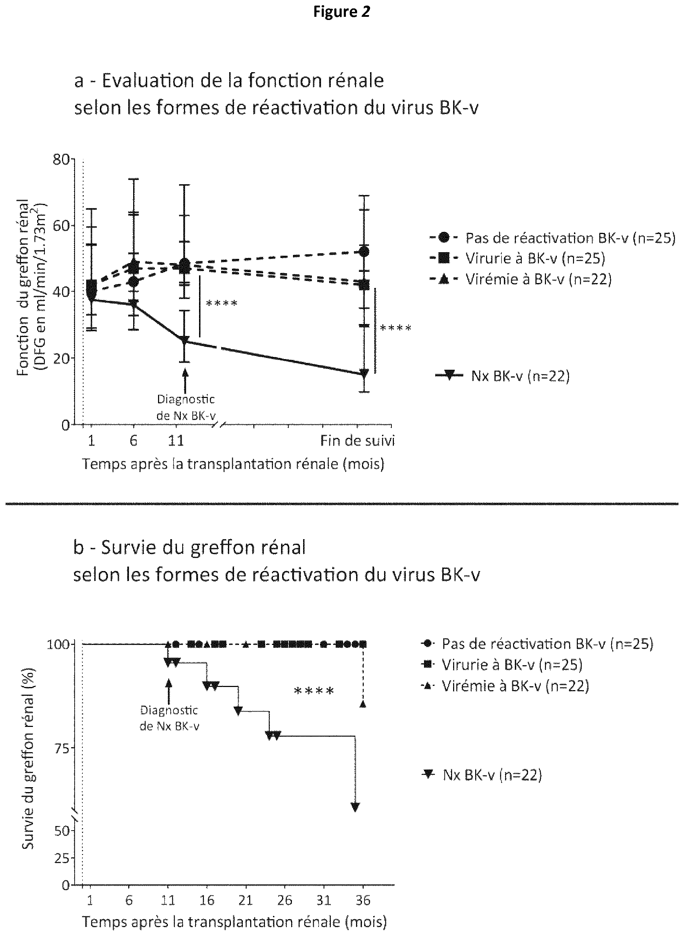

La figure 2 montre l'évaluation de la fonction rénale (par mesure du débit de filtration glomérulaire - partie a) et le taux de survie des greffons rénaux (partie b) à 3 ans post-greffe chez des patients avec ou sans Nx BK-v. Ces données sont issues de l'étude de cohorte ayant permis de développer la présente invention. Cette étude observationnelle (non interventionnelle) a été menée par les présents inventeurs de novembre 2014 à novembre 2017 sur une cohorte de 94 patients transplantés rénaux répartis en 4 groupes selon leur niveau de réactivation BK-v : un groupe sans réactivation BK-v (n=25), un groupe avec virurie BK-v isolée (n=25), un groupe avec virémie BK-v isolée sans Nx BK-v prouvée histologiquement (n=22) et un groupe avec Nx BK-v prouvée histologiquement (n=22). La figure 2 démontre que les patients présentant une Nx BK-v ont une dysfonction sévère du greffon rénal. Cette dysfonction est présente dès le diagnostic de Nx BK-v et s'aggrave progressivement, avec une perte de greffon dans près de 50% des cas après 3 ans de suivi.

Il est donc essentiel de détecter précocement la Nx BK-v chez les patients transplantés rénaux, voire de l'anticiper, pour mieux tenter de l'enrayer.

Facteurs de risques et démarches diagnostiques et thérapeutiques actuelles de la Nx BK-v

Il est connu que la réplication du BK-v est favorisée par différents facteurs dont la nature et l'intensité du traitement immunosuppresseur et l'altération de la réponse immune spécifique du BK-v ([8]). Plus précisément, le risque de développer une Nx BK-v est significativement associé à l'intensité du traitement immunosuppresseur, à l'association tacrolimus-mycophénolate mofétil (traitement de référence actuel en greffe rénale), ainsi qu'à une exposition cumulée importante aux corticostéroïdes (> 3000 mg de corticostéroïdes dans les 6 mois précédents la virémie BK-v). D'autres facteurs de risques non liés aux traitements immunosuppresseurs ont également été identifiés tels que l'âge du donneur et du receveur, le sexe du receveur ou des caractéristiques propres au greffon telles que les lésions d'ischémie-reperfusion ([7]).

Il n'existe à ce jour aucune thérapie antivirale spécifique contre ce virus. Les traitements tels que le Cidofovir sont responsables d'une toxicité importante pour le rein transplanté et sont contre-indiqués en cas de dysfonction sévère du greffon. Par ailleurs, le Léflunomide ou les fluoroquinolones ont pu se révéler efficaces dans certains cas isolés. Cependant, l'efficacité inconstante et les effectifs insuffisants de ces rares cas ne permettent pas de recommander actuellement l'utilisation en pratique courante de ces molécules [24].

Le seul traitement actuellement reconnu pour traiter la Nx BK-v est l'allègement de l'immunosuppression thérapeutique à laquelle le patient receveur du greffon est soumis ([7], [24]). En effet, la réactivation du BK-v survient lorsque l'activité du système immunitaire est réduite par le traitement immunosuppresseur. Lorsqu'une Nx BK-v est détectée, le premier réflexe thérapeutique est de réduire l'immunosuppression. Cet ajustement thérapeutique est cependant fait de façon empirique, sans limite quantitative, exposant alors le patient à un risque de rejet allogénique et de perte de greffon. Il est donc important de n'avoir recours à cette approche thérapeutique que dans les cas où le patient souffre effectivement d'une Nx BK-v ou est considéré à très haut risque d'en développer une, et ce de manière progressive et contrôlée. Le traitement immunosuppresseur devra ensuite être ré-augmenté pour ne pas accroitre les risques de rejet du greffon. Il est important également de ne pas réduire l'immunosuppression chez des patients souffrant simplement de virémie ou de virurie isolées à BK-v, car cette diminution mettrait en péril le greffon, alors que celui-ci n'est pas infecté par le BK-v.

Une infection à BK-v peut être facilement détectée par PCR quantitative sur un échantillon d'urine ou de sang. Cependant, comme expliqué ci-dessus, la détection de BK-v dans le sang ou les urines des patients transplantés n'est pas nécessairement associée à un mauvais pronostic pour le greffon, puisqu'un grand nombre de patients virémiques ou viruriques ne développeront pas de Nx BK-v (la réactivation virale n'atteignant pas toujours le greffon lui-même). En effet, si une charge virale du BK-v $\geq 10^4$ copies/mL de sang est associé à une sensibilité de plus de 90%, ce test n'est pas suffisamment performant pour le diagnostic de Nx BK-v, sa valeur prédictive positive ne dépassant pas 55% ([1], [25], [26]).

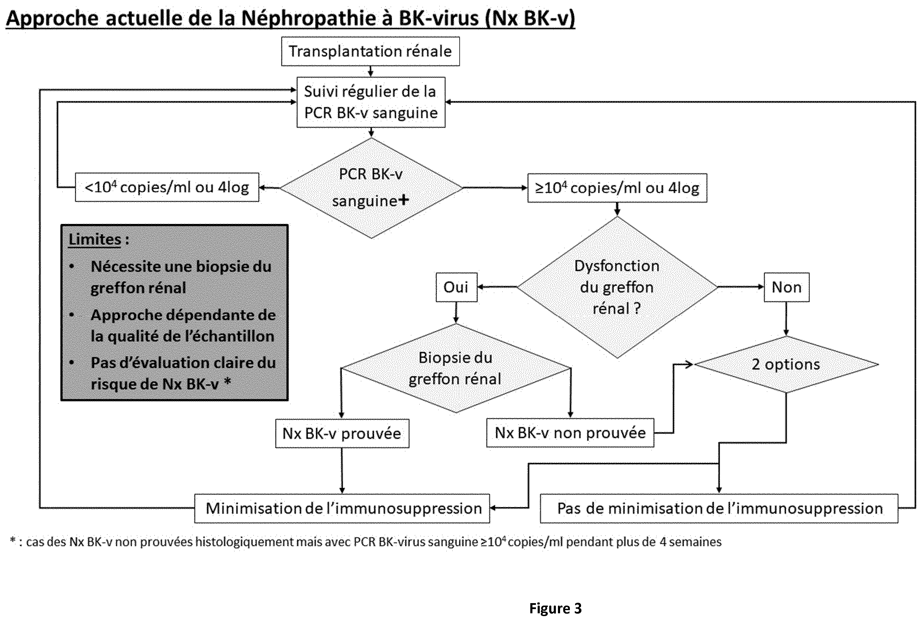

A ce jour, la diminution du traitement immunosuppresseur est réalisée en cas de Nx BK-v prouvée ou suspectée (PCR BK-v positive pendant au moins 4 semaines et $\geq 10^4$ copies/mL de sang). Cependant, cette solution n'est pas optimale puisque seront considérés « à risque » des patients présentant une simple virémie isolée, sans atteinte du greffon rénal. La figure 3 illustre cette problématique.

L'analyse histologique du parenchyme rénal après biopsie du greffon rénal constitue aujourd'hui la seule méthode fiable pour diagnostiquer une Nx BK-v. Cependant, il s'agit d'un acte invasif à risque de complications hémorragiques et pouvant être faussement négatif dans 10 à 30 % des cas, en particulier durant les stades précoces de Nx BK-v. En outre, en l'absence de dégradation de la fonction du greffon rénal, il n'est pas recommandé de faire subir à ces patients des biopsies répétées pour détecter ou confirmer la présence de lésions produites par le BK-v ([7]). Ainsi, la démarche diagnostique de la Nx BK-v actuellement recommandée repose sur le suivi longitudinal de la charge virale du BK-v dans le sang du patient, et la réalisation d'une biopsie du greffon rénal en cas de dégradation de la fonction du greffon et/ou d'une charge virale BK-v $\geq 10^4$ copies/mL de sang pendant au moins 4 semaines consécutives. Cependant, l'altération de la fonction du greffon évaluée par le débit de filtration glomérulaire est dans le cas du diagnostic de la Nx BK-v un marqueur tardif, associé à une extension déjà importante des lésions fibrosantes. Ces atteintes irréversibles sont associées à un risque important de perte de greffon (cf. tableau 8 de Hirsch et al, 2014 [7] et figure 2).

Ainsi, la stratégie actuelle est à l'origine de retards et de difficultés diagnostiques alors que la prise en charge thérapeutique de cette complication doit être la plus précoce possible afin d'éviter la survenue de lésions irréversibles.

D'autres tests diagnostiques de la Nx BK-v ont été proposés par le passé. Parmi eux, le monitorage immuno-virologique est basé sur l'étude des capacités fonctionnelles des lymphocytes T totaux ou anti-viraux et vise à identifier les patients les plus à risque de complications virales ([12]). Certains auteurs ont étudié la réponse immune spécifique anti BK-v pour identifier les patients à risque de Nx BK-v ([8], [9], [27]). Une faible réponse immune spécifique anti BK-v, évaluée par les capacités de sécrétion d'Interféron-$\gamma$ des lymphocytes T stimulés par des antigènes du BK-v (tests de type ELISA ou ELISPOT), a été retrouvée chez les patients avec réactivation sanguine BK-v ([22], [23]). D'autres équipes ont utilisé des tests d'évaluation globale de la réponse immune lymphocytaire tel que le « Cylex ImmunKnow Test » dans le cadre de réactivations sanguines et urinaires du BK-v ([20]). Dans cette étude, les auteurs [20] ne mettaient pas en évidence d'association entre la réponse lymphocytaire globale et la Nx BK-v mais uniquement avec la virémie. Par ailleurs, certains auteurs ont proposé différentes techniques individuelles d'évaluation du risque de Nx BK-v, telles que la mesure de la charge virale BK-v ([25], [30]) ou la réponse lymphocytaire anti BK-v ([8]). Ces documents ne proposent pas de combiner différents paramètres ni d'appliquer un paramètre de normalisation permettant l'obtention d'un index de prédiction de risque de Nx BK-v. Ainsi, ces méthodes n'ont jamais permis ni de diagnostiquer la Nx BK-v ni d'identifier de manière fiable les patients à risque de développer une Nx BK-v. La recherche de meilleurs paramètres est toujours en cours ([21]). A ce jour, seule une histologie réalisée sur une biopsie rénale permet de diagnostiquer objectivement une Nx BK-v ([7], [9]).

Ainsi, il existe un besoin urgent de diagnostiquer de manière fiable, précoce et non invasive les patients atteints de Nx BK-v, ainsi que de stratifier le risque de développer cette complication chez les patients ayant subi une transplantation rénale. Ces méthodes diagnostiques et pronostiques devront idéalement discriminer les patients à risque de développer une Nx BK-v de ceux ayant une virémie isolée du BK-v, sans atteinte du greffon ni risque ultérieur de complication de ce type.

Description de l'invention

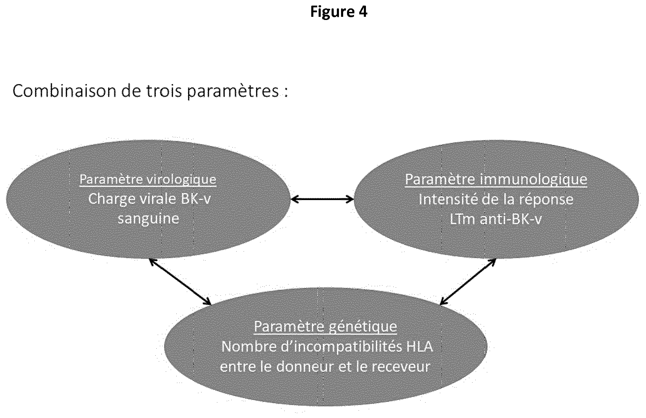

La présente invention répond à ce besoin, en fournissant une méthode non invasive et précoce d'évaluation personnalisée du risque de Nx BK-v. L'originalité de la méthode de l'invention est basée sur la prise en compte d'une combinaison de trois paramètres spécifiques au patient, qui sont i) un paramètre virologique évalué par la charge virale du BK-v dans le sang du patient, ii) un paramètre immunologique évalué par l'intensité de la réponse lymphocytaire T mémoire anti-BK-v (réponse LTm anti-BK-v) chez le patient, et iii) le nombre d'incompatibilités HLA dans le couple [donneur/receveur] (figure 4). Ces paramètres doivent être combinés pour obtenir un index complet, fiable et reproductible, permettant un suivi individualisé, à un moment t donné, des patients transplantés rénaux. Cette méthode est plus fiable que les tests de l'art antérieur, puisque les patients souffrant d'une virémie isolée à BK-v ne seront pas classés « à risque » (sur 27 patients testés, sa valeur prédictive positive est de 100% et le taux de faux positif est nul, cf. les exemples ci-dessous). De plus, l'utilisation du marqueur lié aux lymphocytes T mémoires anti-BK-v (LTm anti-BK-v) permet une évaluation précoce du risque de Nx BK-v, avant l'apparition de lésions irréversibles du parenchyme rénal. Par-dessus tout, cette méthode est peu invasive (de simples prises de sang sont requises), et peut donc être reproduite à différents moments après la greffe, sans risque pour le patient. Grâce à cette répétabilité potentielle, la méthode de l'invention permet de mieux évaluer la réponse à la prise en charge thérapeutique et *in fine* aider à moduler l'immunosuppression thérapeutique ou tout autre traitement qui permettrait de juguler la réactivation BK-v, le tout sans avoir recours à une biopsie du greffon rénal.

## Description détaillée de l'invention

La méthode de l'invention est basée sur la prise en compte de trois facteurs, un paramètre virologique, un paramètre immunologique et un paramètre génétique, qui doivent être combinés entre eux. Ceci permet d'obtenir un index complet, fiable et précoce du risque de développer une Nx BK-v pour un patient donné. Ces facteurs sont :

i) la charge virale du virus BK-v dans le sang du patient à un temps donné,

ii) l'intensité de la réponse LTm anti-BK-v de ce même patient au même temps,

iii) le nombre d'incompatibilités des molécules HLA entre le donneur du greffon et le patient / receveur, déterminé au moment de la greffe de rein.

La figure 4 illustre la méthode de l'invention basée sur la combinaison de ces trois paramètres.
La combinaison de ces marqueurs permet d'évaluer individuellement, à un temps t donné, le risque de survenue d'une Nx BK-v pour le patient testé. Cette méthode permet ainsi un suivi individualisé des patients transplantés rénaux.
La présente méthode dispose des avantages suivants par rapports aux analyses proposées dans l'art antérieur :

➢ Multiparamétrique, elle intègre la charge virale BK-v, l'intensité de la réponse LTm anti-BK-v et les différences d'allèles HLA au sein du couple donneur/receveur et permet ainsi une évaluation plus complète de la situation immuno-virologique de chaque patient.
➢ Elle est rapide, peu couteuse, standardisable et fiable, permettant un suivi prospectif du patient, pouvant être répété à intervalle de temps régulier en fonction de l'état du patient, du traitement administré, et des résultats précédemment obtenus.
➢ Elle est non-invasive puisqu'elle ne requiert qu'un échantillon de 15ml de sang du patient.
➢ Elle permet de détecter les patients à risque de Nx BK-v de manière plus précoce que les méthodes de l'art antérieur, notamment celles basées sur la sécrétion cytokinique des lymphocytes T spécifiques du BK-v.
➢ Son résultat est une valeur absolue, normée, qui peut être comparée entre les patients et au cours de la vie d'un même patient.

Dans un premier aspect, la présente invention concerne donc une méthode pour évaluer le risque de développer une Nx BK-v pour un patient ayant subi une transplantation rénale, ladite méthode comprenant les étapes suivantes :

a) mesurer la charge virale de BK-v dans un échantillon biologique dudit patient,
b) mesurer l'intensité normalisée de la réponse des lymphocytes T CD4+ ou CD8+ mémoires spécifiques du virus BK-v dans un échantillon biologique dudit patient, ladite normalisation étant effectuée par rapport à la charge virale BK-v mesurée à l'étape a), et
c) déterminer les différences d'allèles HLA entre le donneur de rein et ledit patient, de préférence le nombre d'incompatibilités entre les HLA I et HLA II du donneur de rein et dudit patient,

d) évaluer le risque de développer une néphropathie à BK virus (BK-v) chez un patient ayant subi une transplantation rénale en combinant les paramètres déterminés aux étapes a), b) et c).

Cette méthode permet, une fois que l'ensemble des paramètres a), b) et c) mentionnés ci-dessus ont été collectés et combinés, d'aider à diagnostiquer la Nx BK-v ou à stratifier le risque de développer une Nx BK-v. Ces étapes peuvent être effectuées dans n'importe quel ordre. Ceci étant dit, l'étape c) a été réalisée au préalable des étapes a) et b), le nombre d'incompatibilités HLA étant connu au moment de la transplantation rénale.

L'échantillon biologique utilisé dans l'étape a) est par exemple un échantillon de sang total ou de plasma tandis que l'échantillon biologique utilisé dans l'étape b) est de préférence un échantillon de sang total. Dans un mode de réalisation où les étapes a) et b) sont réalisées par deux laboratoires indépendants, deux échantillons distincts peuvent être utilisés pour les mesures des étapes a) et b), par exemple deux échantillons de sang prélevés indépendamment l'un de l'autre. Dans le cas où deux échantillons distincts sont utilisés, ceux-ci ont été prélevés de préférence à faible intervalle de temps, typiquement dans la même journée, par exemple à quelques heures d'intervalle (entre 1h et 4h), voire dans la même heure.

Dans un mode de réalisation préféré, les étapes a) et b) sont réalisées par le même laboratoire, avec un même échantillon biologique utilisé pour les mesures des étapes a) et b). Dans un mode de réalisation encore plus préféré, cet échantillon est un échantillon de sang, prélevé de manière non invasive, indolore, par prise de sang intraveineuse classique. Cet échantillon de sang peut être analysé directement ou traité comme proposé ci-après au regard de la nature des mesures à effectuer. La quantification de la virémie BK-v (exprimée en nombre de copies/mL de sang) peut se faire par PCR quantitative à partir du sang total ou sur du plasma obtenu par centrifugation à partir du sang du patient. Les cellules mononuclées du sang total (PBMCs), comprenant les LTm anti-BK-v, peuvent quant à elles être isolées par gradient de Ficoll à partir de l'échantillon sanguin du patient. Alternativement, il est possible de lyser les globules rouges présents dans le sang du patient, et de détecter la présence des LT par cytométrie de flux (marqueurs FSC-A et SSC-A), puis de mesurer la prolifération des LTm anti-BK-v soumis à une stimulation au BK-v en utilisant le marqueur de prolifération cellulaire Ki67.

Le « patient » est, dans le cadre de la présente demande, un individu, humain ou animal, ayant eu une transplantation rénale au cours de son existence. Dans un mode de réalisation préféré, ladite transplantation a eu lieu dans les 5 dernières années précédant le prélèvement des échantillons. Dans un mode de réalisation encore plus préféré, ladite transplantation a eu lieu dans les 3 dernières années précédant le prélèvement des échantillons. Dans un mode de réalisation préféré entre tous, ladite transplantation a eu lieu dans les 24 mois précédant le prélèvement des échantillons.

Dans un mode de réalisation préféré, la méthode de l'invention est utilisée pour diagnostiquer ou pronostiquer une Nx BK-v chez un patient faisant une réactivation sanguine à BK-v. De manière préférentielle, la méthode de l'invention s'applique à des patients dont la charge virale de BK-v est supérieure à 200 copies/mL dans le sang.

En ne considérant dans un premier temps que les patients transplantés rénaux avec virémie BK-v, cela représente près de 840 patients par an en France (pour près de 3500 nouvelles transplantations rénales annuelles) et 7200 patients par an en Europe et aux USA (pour plus de 30000 nouvelles transplantations rénales annuelles).

La méthode de l'invention peut également s'appliquer aux patients traités pour des rejets de greffes et/ou à risque de développer ultérieurement une Nx BK-v (entre 15 et 20% de l'ensemble des patients transplantés rénaux, soit entre 5500 patients en France et 12000 en Europe et aux USA), ainsi qu'à ceux présentant une virémie BK-v et/ou une dégradation inexpliquée de la fonction rénale au-delà de la 1ère année de transplantation.

Ainsi, près de 6340 patients transplantés rénaux en France et plus de 19200 en Europe et aux USA pourraient être concernés chaque année par la méthode. La présente méthode pourra aussi être reproduite périodiquement pour le suivi d'un même patient.

Les trois étapes de la méthode de l'invention sont détaillées ci-après.

### a) Etape a : Mesure de la charge virale BK-v dans le sang du patient

Cette étape consiste à mesurer, à partir d'un échantillon de sang total ou de plasma du patient, le nombre de copies de BK-v par mL de sang. Ce nombre de copies de BK-v peut être quantifié selon les méthodes classiques décrites dans l'art. Typiquement, cette mesure peut être obtenue en utilisant des techniques de PCR, telles que la PCR quantitative.

#### • Evaluation de la charge virale BK-v

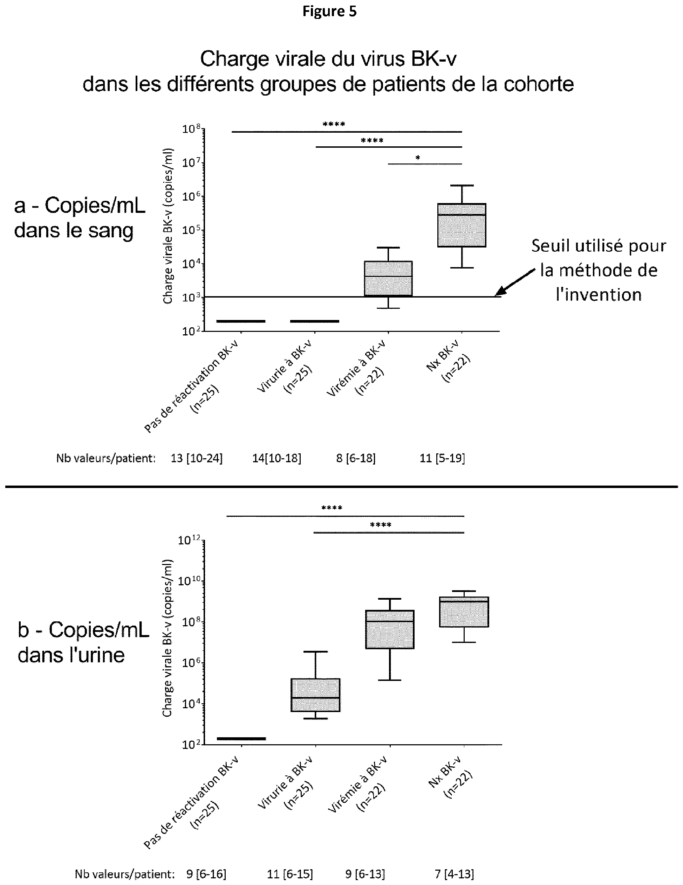

La mesure de la charge virale BK-v par ml de sang et/ou d'urine d'un patient est réalisée de manière systématique pour l'ensemble des patients transplantés rénaux selon les recommandations internationales. Néanmoins, il est important de rappeler qu'une mesure isolée de la charge virale BK-v dans le sang et/ou dans les urines d'un patient ne saurait être, à elle seule, le marqueur diagnostique d'une Nx BK-v, puisque de nombreux patients souffrant de virémie ou de virurie à BK-v n'ont pas de Nx BK-v (figure 1, [9]). Ainsi, cette mesure n'a pas pour vocation d'être utilisée, en tant que telle, comme marqueur diagnostique de Nx BK-v, comme certaines équipes ont pu le proposer ([10]). Cette dernière étude ([10]), réalisée sur un faible nombre de patients, n'a pas été confirmée sur une population plus large. De plus, la charge virale BK-v sanguine ne permet pas de distinguer de façon fiable les patients souffrant de Nx BK-v de ceux souffrant de virémie à BK-v isolée. En effet, une récente méta-analyse a mis en évidence qu'une charge virale de BK-v $\geq 10^4$ copies/mL de sang était associée à une valeur prédictive positive de 55% pour le diagnostic de Nx BK-v ([26]). La figure 5 illustre cette notion en montrant les différents niveaux de charges virales sanguines et urinaires du BK-v mis en évidence par les inventeurs pour élaborer la présente méthode. De plus, en accord avec l'état de l'art antérieur, il est important de noter que si une charge virale sanguine du BK-v > $10^5$ peut être associé à une Nx BK-v avec une sensibilité de près de 100% ([26]), ce paramètre est tardif car déjà associé à des lésions du parenchyme rénal et à une dysfonction chronique sévère du greffon au moment du diagnostic de Nx BK-v (figure 2a). Par ailleurs, du fait de l'absence de différence significative entre les patients avec virémie BK-v isolée et Nx BK-v, la charge virale BK-v urinaire n'a pas été prise en compte dans l'élaboration de l'invention (figure 5b).

Dans un mode de réalisation préféré, l'échantillon biologique utilisé pour l'étape a) est un échantillon de sang total (incluant le plasma et les éléments figurés du sang, à savoir les globules rouges, les leucocytes et les plaquettes) ou un échantillon de plasma.

Cet échantillon de sang total ou de plasma peut être traité selon les techniques classiques dans l'art.

Dans un mode de réalisation encore plus préféré, la charge virale de BK-v est mesurée à l'étape a) par PCR quantitative à partir d'un échantillon biologique (par exemple de sang total ou de plasma) dudit patient.

• Normalisation de la charge virale BK-v sanguine

Dans le cadre de l'invention, la mesure de la charge virale BK-v sanguine permet uniquement de quantifier la quantité de virus circulant dans le sang du patient à l'instant de la réalisation du test de l'invention. Cette valeur de charge virale mesurée à l'étape a) permet de normaliser l'intensité de la réponse LTm anti-BK-v obtenue à l'étape b) afin d'obtenir un index indépendant et comparable entre individus. Les inventeurs ne l'utilisent pas comme marqueur pronostic, comme proposé dans l'art antérieur, en comparant la charge virale à un seuil (par exemple, $4\log_{10}$ copies d'ADN/mL pendant au minimum 4 semaines) ([9]).

Plus précisément, cette étape permet de révéler si l'infection virale est absente, présente ou anormalement élevée dans le sang du patient, de sorte à pondérer les résultats obtenus à l'étape b) concernant l'intensité de la réponse LTm anti-BK-v chez ledit patient, au moment du prélèvement.

Dans un mode de réalisation préféré de l'invention, il est possible de normaliser le paramètre b) de la méthode de l'invention pour une valeur fixe de copies de BK-v par mL de sang. A partir des résultats ayant permis l'élaboration de la présente méthode, la valeur fixe de normalisation de $10^3$ copies de BK-v par mL de sang a été intégrée comme paramètre virologique dans le calcul de la méthode de l'invention. Cette valeur de normalisation de $10^3$ copies de BK-v a été définie afin de permettre une évaluation du risque de Nx BK-v la plus précoce possible et ce, dès le stade de virémie à BK-v isolée (figure 5). Les seuils plus élevés de copies de BK-v étaient plus tardifs, associés à des diagnostics de Nx BK-v (figures 5a) et à des lésions du parenchyme rénal (figures 2a).

Néanmoins, ce seuil n'étant qu'un seuil de normalisation fixé par les inventeurs, d'autres valeurs peuvent être utilisées pour normaliser le paramètre b), en fonction du patient, du groupe de patient, etc.

La valeur seuil de positivité de la charge virale BK-v est fixée à 200 copies/mL de sang et on peut, par exemple, utiliser comme seuil de normalisation $3.7\log_{10}$, $4\log_{10}$ ou $4.2\log_{10}$ (seuils précédemment proposés dans la littérature ([26]).

Une fois normalisé, le résultat obtenu à la mesure de l'étape b) pourra ainsi être comparé entre individus, ou, à différents intervalles de temps pour un même individu. Cette comparaison inter-individuelle permettra de classer le patient suivant le niveau de risque de son groupe tandis que la comparaison intra-individu permettra de suivre l'évolution au cours du temps du niveau de risque de développer une Nx BK-v chez un même patient.

**b) Etape b : Evaluation de l'intensité normalisée de la réponse lymphocytaire T mémoire anti-BK-v**

Cette étape consiste à évaluer, à partir d'un échantillon de sang périphérique, la réponse LTm anti-BK-v à un temps t donné en mesurant la capacité de prolifération lymphocytaire en réponse à une stimulation par des peptides spécifiques du BK-v. La normalisation de cette réponse immune spécifique par rapport à la charge virale BKv dans le sang permet d'obtenir l'intensité normalisée de la réponse LTm anti-BK-v, permettant ainsi des comparaisons intra et inter individus.

• Evaluation de la réponse LTm anti-BK-v

Les lymphocytes T mémoires anti-BK-v (LTm anti-BK-v) sont des lymphocytes T possédant des immunorécepteurs (TCR) spécifiques d'antigènes du BK-v et ayant été activés au préalable par un contact antérieur avec le BK-v. Ces lymphocytes gardent la « mémoire » de leur rencontre avec ce virus au cours de la primo-infection virale. Cette mémoire leur permet de mieux réagir lors d'un nouveau contact avec ce virus, en développant une réponse secondaire rapide et efficace. Il est important de noter que la séroprévalence des polyomavirus humains est très élevée au niveau mondial (80 - 90 % à l'âge adulte pour le BK-v et le JC-v), témoignant d'une exposition précoce et large à ces virus.

Les performances de cette réponse secondaire ne sont pas simplement le fait d'une augmentation quantitative des LTm anti-BK-v. A la plus grande fréquence des clones spécifiques immédiatement recrutables au moment d'un nouveau contact antigénique, s'ajoute une meilleure fonctionnalité des cellules « mémoires » quand on les compare à celles de cellules naïves, en termes de capacités prolifératives, de sécrétion de cytokines et de cytotoxicité. Ces performances accrues des lymphocytes T mémoires sont liées à des modifications épigénétiques au niveau des promoteurs des gènes impliqués dans la réponse immune.

L'étape b) de la méthode de l'invention consiste donc à évaluer l'intensité de la réponse LTm anti-BK-v présents dans l'échantillon biologique du patient testé en mesurant la capacité de prolifération des LTm anti-BK-v en réponse à une stimulation par des peptides spécifiques du BK-v et en normalisant cette réponse à une charge virale fixe de BK-v (intensité normalisée).

La capacité proliférative est un élément pivot de la polyfonctionnalité des réponses lymphocytaires T CD4 et T CD8 mémoires et une des premières fonctionnalités perdues au cours des dysfonctionnements immuns lors des infections virales chroniques. La capacité proliférative traduit la richesse des populations T CD8 mémoires en cellules de type $T_{CM}$ et $T_{SCM}$ (cellules T centrales mémoires et cellules souches mémoires). Ces cellules ont des capacités élevées de prolifération, d'auto-renouvellement et de différentiation en cellules effectrices. Par ailleurs elles fournissent des signaux d'aide indispensables à une fonctionnalité optimale des cellules effectrices.

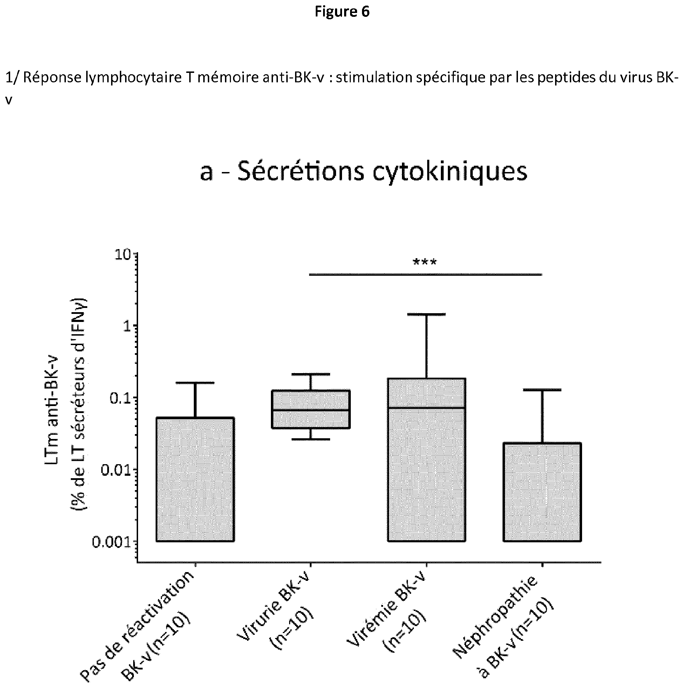

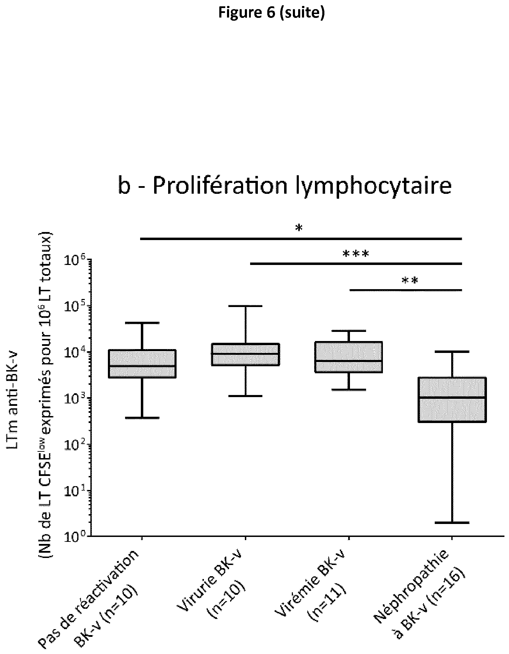

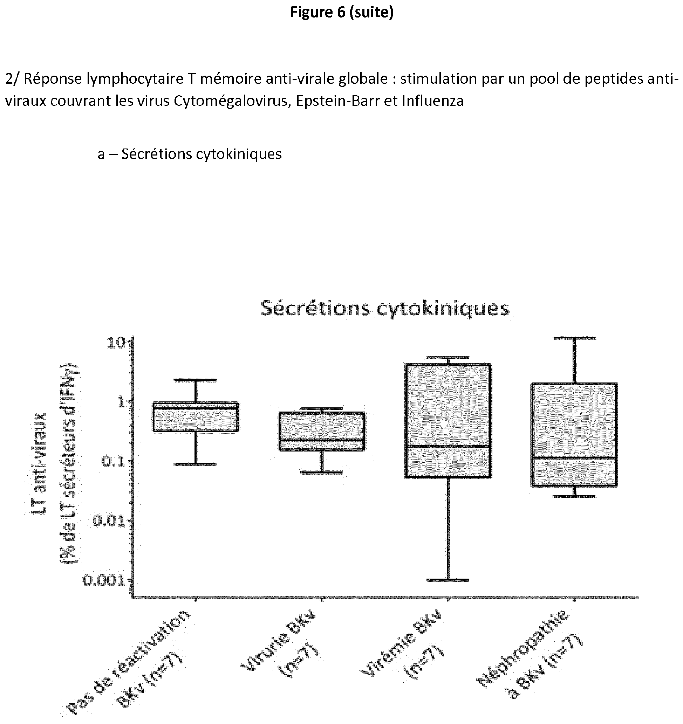

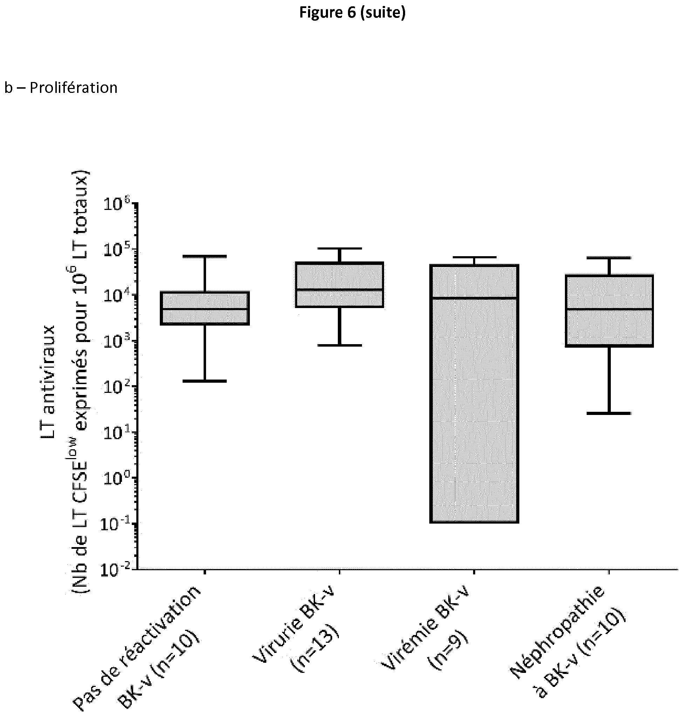

Il ne s'agit pas, comme proposé dans l'art antérieur, de mesurer la sécrétion cytokinique des lymphocytes T contenus dans le sang des patients, en présence de peptides du BK-v ([9]). Plus précisément, il ne s'agit pas non plus de mesurer la sécrétion d'interféron gamma en réponse à une stimulation peptidique d'antigènes du BK-v par des techniques de type ELISA ou ELISPOT, comme proposé dans [22], et encore moins avant et après la greffe comme proposé dans [23]. Ces techniques de l'art antérieur ne permettent pas une évaluation précise des capacités de reconstitution de l'immunité mémoire. En effet, la capacité de sécrétion d'Interféron-y permet d'étudier préférentiellement des sous-populations lymphocytaires mémoires dites « terminales » de type effecteurs mémoires ou effecteurs terminaux. Ces sous-populations lymphocytaires très différenciées sont peu, voire non dotées de capacités d'auto-renouvellement, de prolifération et de différentiation lymphocytaire et ne permettent donc pas d'évaluer de façon optimale les capacités de reconstitution de l'immunité mémoire antivirale ([13], [14]). En se basant sur les résultats ayant permis l'élaboration de la présente méthode, les inventeurs ont pu démontrer que l'évaluation seule de la sécrétion cytokinique d'Interféron-y n'est pas le paramètre de fonctionnalité lymphocytaire le plus pertinent pour discriminer de manière fiable et reproductible les patients avec virémie BK-v des patients avec Nx BK-v (figure 6/1a).

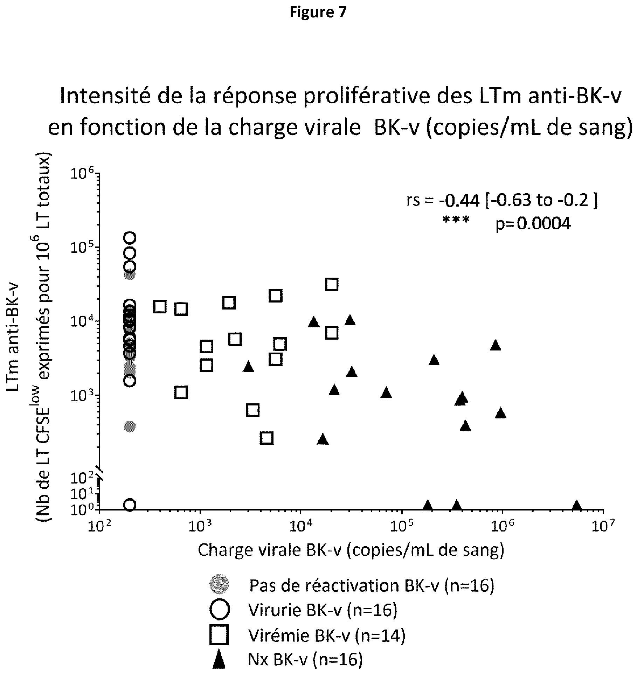

La prolifération lymphocytaire est la méthode d'évaluation la plus discriminante (figure 6/1b). En effet, la réponse proliférative était significativement plus faible chez les patients avec Nx BK-v par rapport aux 3 autres groupes de patients (sans réactivation BK-v, virurie BK-v et virémie BK-v isolée (figures 6/1b)). Ceci n'était pas le cas en évaluant la sécrétion d'Interféron-y, où la réponse cytokinique était significativement plus faible entre les patients avec Nx BK-v et virurie BK-v, mais pas entre les patients avec Nx BK-v et virémie BK-v (figures 6/1a). Par ailleurs, l'intensité de la réponse proliférative des LTm anti-BK-v (évaluée par le nombre de lymphocytes T CFSE[low] (cf infra)) était négativement corrélée à la charge virale de BK-v dans le sang (figure 7).

Les présents inventeurs ont donc privilégié l'évaluation de la prolifération lymphocytaire par rapport aux autres techniques précédemment employées pour évaluer les capacités de reconstitution de l'immunité mémoire spécifique du BK-v et élaborer la méthode de l'invention.

• Identification de la réponse LTm anti-BK-v

Dans un mode de réalisation préféré, l'échantillon biologique utilisé dans l'étape b) est un échantillon de sang total. Ledit échantillon biologique est, de manière encore plus préférée, un échantillon de sang total prélevé sur le patient de manière indolore par ponction intraveineuse périphérique.

Comme expliqué ci-dessus, les lymphocytes T mémoires sont naturellement présents dans ce type d'échantillon biologique si le patient a été infecté par le virus BK-v. Ces lymphocytes mémoires vont réagir rapidement lorsqu'ils sont (re) mis en contact avec des antigènes du virus BK-v.

Dans un mode de réalisation particulier, les cellules mononucléées présentes dans l'échantillon biologique sont isolées selon les méthodes classiques de purification cellulaire. Elles sont séparées des autres composantes de l'échantillon par leur densité, en utilisant des gradients de Ficoll-Hypaque (ou équivalents). Après centrifugation, les cellules de faible densité (lymphocytes, monocytes/macrophages, cellules dendritiques et autres cellules présentatrices d'antigènes) sont en suspension au-dessus du Ficoll alors que toutes les autres composantes de l'échantillon forment un culot. Les cellules mononuclées peuvent alors être récupérées à l'aide d'une pipette.

Dans un mode de réalisation plus particulier, les cellules mononuclées du sang total sont ensuite stimulées par un mélange d'antigènes de virus BK-v (par exemple Peptivator® BKV VP1 ou Peptivator® BKV LT commercialisés par Myltenyi Biotec®, ou équivalents, à la concentration finale de 1 μg/mL/peptide). De préférence, des mélanges de peptides BK-v chevauchants sont utilisés afin de s'affranchir des différences HLA entre patients. Les LTm anti-BK-v présents dans l'échantillon vont alors proliférer activement au contact de ces antigènes. Cette stimulation peut durer quelques jours (typiquement de 4 à 7 jours, de préférence 5 jours).

Ainsi, la réponse LTm anti-BK-v CD4$^+$ ou CD8$^+$ mesurée à l'étape b) de la méthode de l'invention est déterminée de préférence en mettant en contact des cellules mononuclées du sang périphérique dudit patient avec des peptides du virus BK-v, et en évaluant la prolifération des lymphocytes T présents dans lesdites cellules mononuclées après quelques jours de culture (typiquement de 4 à 7 jours, de préférence 5 jours de culture). Cette prolifération peut être mesurée en détectant l'expression d'un marqueur de division cellulaire (type CFSE ou équivalent) dont l'intensité diminue au cours des divisions cellulaires, chaque cellule fille contenant la moitié du marqueur contenu dans la cellule mère. Afin d'identifier spécifiquement les LTm anti-BK-v ayant activement proliféré, cette prolifération lymphocytaire est soustraite à la prolifération spontanée des lymphocytes T dudit patient (lymphocytes T ayant été mis en culture dans les mêmes conditions, pendant la même durée mais sans les peptides du BK-v).

De manière encore plus préférée, ladite prolifération spécifique des LTm anti-BK-v est mesurée en déterminant la proportion de lymphocytes T CD4$^+$ ou CD8$^+$ ayant dilué le CFSE (ou un marqueur de division cellulaire équivalent), présents après quelques jours de culture en présence desdits peptides (typiquement de 4 à 7 jours, de préférence 5 jours de culture), et en lui soustrayant la proportion de lymphocytes T CD4$^+$ ou CD8$^+$ ayant dilué le CFSE (ou un marqueur de division cellulaire équivalent), pendant le même temps, mais en l'absence desdits peptides.

De manière encore plus préférée, ladite prolifération spécifique des LTm anti-BK-v CD4$^+$ est mesurée en comparant la proportion de lymphocytes T CD4$^+$ ayant dilué le CFSE (ou un marqueur de division cellulaire équivalent) dans la culture en présence des peptides, avec la proportion desdits lymphocytes ayant dilués le même marqueur et cultivés dans les mêmes conditions de culture, mais sans lesdits peptides.

De manière encore plus préférée, ladite prolifération spécifique des LTm anti-BK-v CD8$^+$ est mesurée en comparant la proportion de lymphocytes T CD8$^+$ ayant dilué le CFSE (ou un marqueur de division cellulaire équivalent) dans la culture en présence des peptides, avec la proportion desdits lymphocytes ayant dilués le même marqueur et cultivés dans les mêmes conditions de culture, mais sans lesdits peptides.

L'intensité de la réponse LTm anti-BK-v peut être mesurée en utilisant n'importe quel marqueur de prolifération cellulaire classiquement utilisé (CFSE, $^3$TH, BRDU ou équivalent, etc).

Les lymphocytes T CD4$^+$ et CD8$^+$ peuvent ensuite être identifiés par cytométrie de flux à l'aide d'anticorps anti-CD4 et anti-CD8 commerciaux.

Dans un mode de réalisation préféré, l'étape b) utilise des cellules mononuclées du sang périphérique purifiées selon toute méthode classique connue dans l'art (par exemple en utilisant le milieu de séparation des cellules sanguines de PAN BIOTECH, ref. P04-60505).

Les lymphocytes T CD4$^+$ et/ou CD8$^+$ ayant proliféré suite à la stimulation antigénique BK-v spécifique peuvent être qualifiés de lymphocytes mémoires « répondeurs » ou de « fonctionnels », puisqu'ils répondent à la stimulation antigénique spécifique et présentent une cinétique de prolifération typique de lymphocytes mémoires.

Après quelques jours de culture (typiquement de 4 à 7 jours, de préférence 5 jours de culture) en présence de peptides spécifiques du BK-v, le test de prolifération renseigne sur le nombre de lymphocytes T mémoires CD4$^+$ ou CD8$^+$ anti-BK-v initialement présents dans l'échantillon du patient.

Après stimulation peptidique spécifique, la proportion de LT ayant dilué le CFSE (LT CFSE$^{low}$) est liée à la présence des LTm anti-BK-v fonctionnels initialement présents dans l'échantillon. La proportion de LT CFSE$^{low}$ mesurée en présence de stimulation peptidique est corrigée par soustraction de la proportion de LT CFSE$^{low}$ obtenue en l'absence des peptides BK-v.

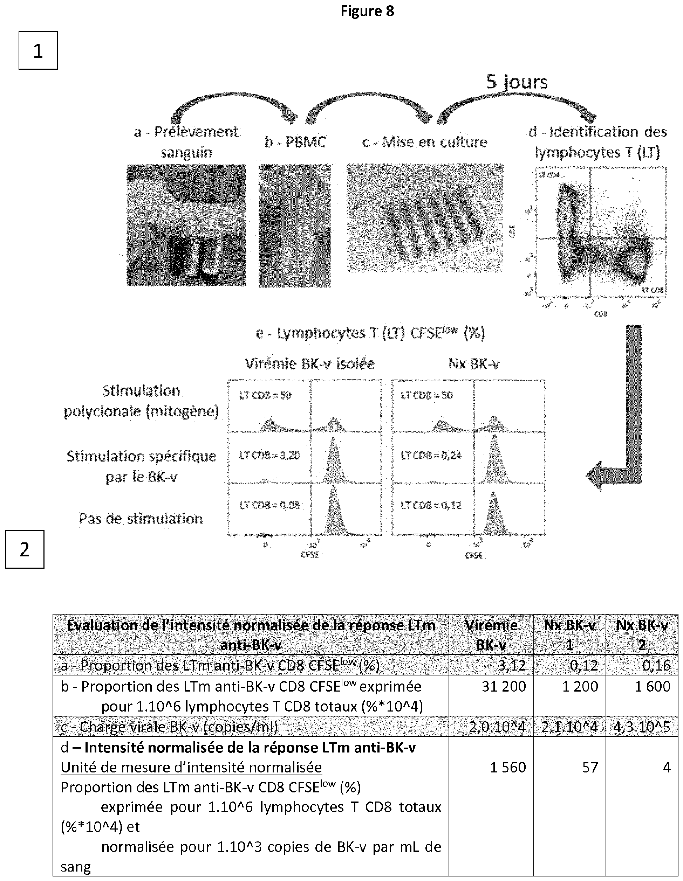

La figure 8/1 illustre le protocole d'identification de la réponse LTm anti-BK-v, via l'analyse de la prolifération des LTm anti-BK-v en réponse à un pool de peptides spécifique du BK-v (proportion de LT CFSE$^{low}$).

• Evaluation de l'intensité normalisée de la réponse LTm anti-BK-v

A partir de la proportion des LTm anti-BK-v CFSE$^{low}$ obtenue plus haut, l'intensité de la réponse LTm anti-BK-v peut être quantifiée en « unité de mesure d'intensité normalisée ».

Une « unité de mesure d'intensité normalisée » est ici définie comme étant la proportion de LTm anti-BK-v CFSE$^{low}$, exprimée pour $10^6$ lymphocytes T totaux et normalisée pour $10^3$ copies de BK-v par mL de sang.

En pratique, on obtient cette « unité de mesure d'intensité normalisée » en multipliant la proportion des LTm anti-BK-v obtenue plus haut par 10 000, puis en normalisant le chiffre ainsi obtenu pour $10^3$ copies de BK-v par mL de sang (en tenant compte de la charge virale réelle du patient testé, telle que mesurée à l'étape a).

Plus explicitement, cette proportion des LTm anti-BK-v CFSE$^{low}$ (exprimée pour $10^6$ lymphocytes T totaux) est normalisée à un nombre fixe de copies de BK-v par mL de sang et reflète ainsi l'intensité normalisée de la réponse LTm anti-BK-v pour une charge virale donnée. Cette normalisation de l'intensité de la réponse LTm anti-BK-v permet une comparaison intra et inter-individus.

La figure 8/2 est un exemple d'évaluation de l'intensité normalisée de la réponse LTm anti-BK-v.

Par exemple,

- si la proportion de LTm anti-BK-v CFSE$^{low}$, mesurée par prolifération lymphocytaire, est de 3,12 % (proportion de LT CFSE$^{low}$ = 3,20 % après stimulation par les peptides BK-v - 0,08 % sans lesdits peptides - figures 8/1e et 8/2a : exemple « Virémie BK-v »).

Ainsi,

- cette proportion de lymphocytes T CFSE$^{low}$ exprimée pour $10^6$ lymphocytes T totaux est de 31 200 (3,12*10000) (figure 8/2b - exemple « Virémie BK-v »)
- et si la charge virale du patient testé est de $2.10^4$ copies de BK-v par mL de sang (figure 8/2c - exemple « Virémie BK-v »),
- alors, l'unité de mesure d'intensité normalisée de la réponse LTm anti-BK-v est de :

$$[31\ 200*1.10^3]/2,0.10^4 = 1\ 560 \text{ (figure 8/2d - exemple « Virémie BK-v »).}$$

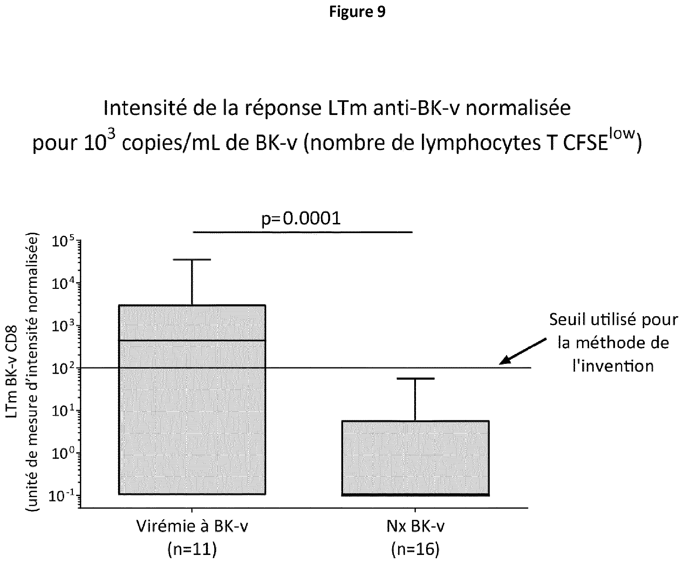

Cette mesure de l'intensité normalisée de la réponse LTm anti-BK-v propre à chaque individu est ensuite comparée à une valeur seuil. A partir des résultats ayant permis l'élaboration de la présente méthode, la valeur seuil de $10^2$ a été intégrée comme paramètre immunologique dans le calcul de la présente méthode. Cette valeur seuil de $10^2$ a été définie en fonction de l'étendue de la distribution de l'intensité normalisée de la réponse LTm anti-BK-v dans le groupe des patients avec Nx BK-v (figure 9).

Plus explicitement, les patients avec Nx BK-v présentaient une intensité normalisée de réponse LTm anti-BK-v plus faible par rapport aux patients avec virémie BK-v isolée (figure 9). La valeur seuil de $10^2$ a été définie dans le groupe des patients avec Nx BK-v en fonction de l'étendue de la distribution non gaussienne de l'intensité de cette réponse afin de prendre en compte jusqu'au 90$^{ème}$ percentile des valeurs de LTm anti-BK-v normalisées (figure 9).

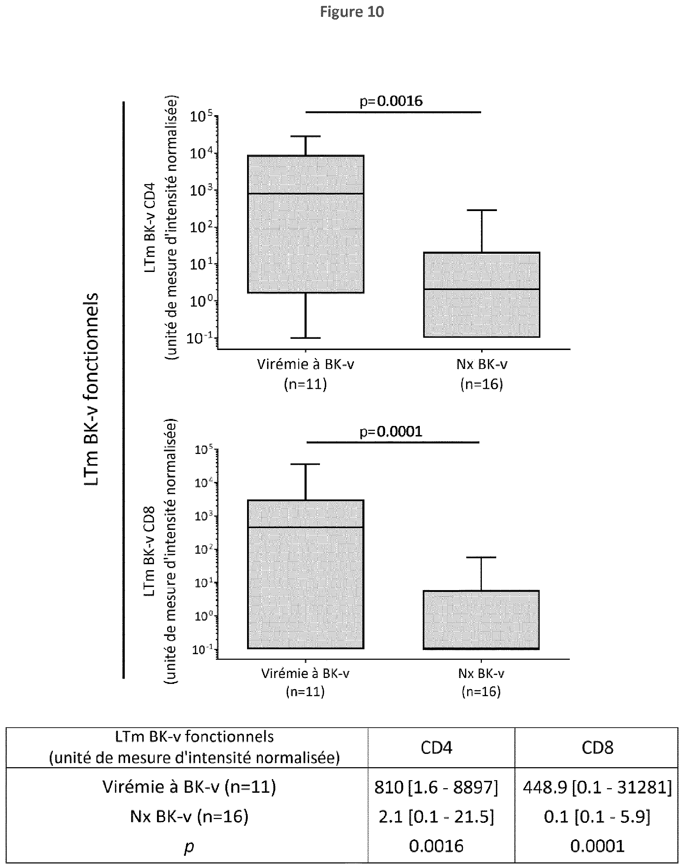

Dans un mode de réalisation préféré de l'invention, seule la prolifération des lymphocytes T CD8$^+$ est mesurée à l'étape b). Sans que cela ne soit limitant, les inventeurs ont pu montrer une meilleure discrimination des patients avec Nx BK-v en se basant sur les lymphocytes T CD8$^+$ par rapport aux lymphocytes T CD4$^+$ (figure 10). Il est possible que cet effet amélioré soit dû à l'importance de l'action directe médiée par les lymphocytes T CD8$^+$ dans le contrôle de la réplication intra-parenchymateuse du BK-v (d'un point de vue immunologique, si les lymphocytes T CD4$^+$ contribuent à l'élaboration et au maintien d'une réponse CD8$^+$ efficace, ce sont en effet les lymphocytes T CD8$^+$ qui vont infiltrer le greffon et détruire les cellules infectées par le BK-v).

Dans ce mode de réalisation, la proportion de lymphocytes T CD8$^+$ CFSE$^{low}$ [mesurée à l'étape b) et exprimée pour $10^6$ lymphocytes T CD8$^+$ totaux] est ainsi normalisée pour $10^3$ copies de BK-v par mL de sang puis comparé au seuil de $10^2$ afin de permettre la classification des patients en fonction du niveau de risque de Nx BK-v, comme expliqué dans la caractérisation de l'index de l'invention ci-dessous.

L'évaluation de la proportion de LTm anti-BK-v CFSE$^{low}$ ainsi que la mesure de la charge virale BK-v seront de préférence répétées au cours de la vie du patient, du fait du caractère évolutif des valeurs de ces paramètres après la transplantation.

### c) Etape c : Mesure du nombre d'incompatibilités HLA entre le donneur et le receveur

Cette étape consiste à mesurer le nombre d'incompatibilités HLA entre le donneur et le receveur du greffon rénal.

• Evaluation du nombre d'incompatibilités HLA entre le donneur et le receveur

Le HLA (*Human Leukocyte Antigens,* antigènes des leucocytes humains) correspond au complexe majeur d'histocompatibilité (CMH). Il désigne des protéines spécialisées (antigènes) présentes sur la surface de toutes les cellules (dont les leucocytes) et les gènes qui codent pour celles-ci. Le CMH assure la fonction de présentation de l'antigène afin de permettre sa reconnaissance par le lymphocyte T par l'intermédiaire de son récepteur spécifique pour l'antigène (TCR). L'extrême diversité (polymorphisme génétique) du CMH en fait un déterminant principal de l'acceptation ou du rejet de la transplantation d'organes entre individus.

Le typage HLA identifie les gènes HLA majeurs de l'individu et les antigènes correspondants qui sont présents sur la surface cellulaire. Ce typage peut être réalisé par des techniques sérologiques ou moléculaires (par PCR).

Pour chaque couple donneur-receveur de greffe, un « nombre d'incompatibilité HLA » est déterminé au moment de la transplantation selon les méthodes classiques décrites dans l'art.

Ce nombre d'incompatibilité est le nombre d'antigènes HLA (HLA-A, -B, -DR, -DQ) différents du donneur par rapport au receveur. Elle est impérativement effectuée lors de la transplantation rénale, car elle garantit en grande partie la réussite de la transplantation. Pour que le risque de rejet de greffe soit minimal, il est important que les antigènes HLA du donneur et du receveur soient les plus proches possible. C'est pourquoi sur l'ensemble des transplantations rénales réalisées en 2016 à partir de donneur décédé, plus de 90% des receveurs avaient moins de 6 incompatibilités HLA avec le donneur [28].

Les règles de calcul des compatibilités HLA sont bien connues. Par exemple, si le donneur est de type HLA-A3 et -A33 et que le receveur est de type HLA-A33 et -A33, le couple donneur / receveur présente 1 incompatibilité dans le HLA-A. Si, par contre, le donneur est de type HLA-A33 et -A33 et que le receveur est de type HLA-A3 et -A33, aucune incompatibilité dans le HLA-A n'est comptabilisée. Cette méthode de calcul des incompatibilités HLA est bien décrite dans la littérature scientifique.

L'évaluation des incompatibilités HLA est généralement réalisée au moment de la transplantation rénale. Il s'agit d'une évaluation fixe (i.e. non évolutive).

L'effet potentiel des incompatibilités HLA sur la réactivation du virus BK-v et le développement des Nx BK-v a été décrit dans quelques études dont les résultats sont discordants (voir notamment dans [29]). Ceci est probablement expliqué par l'hétérogénéité des définitions concernant les différentes réactivations du BK-v dans la littérature (distinction entre les viruries, virémies et Nx BK-v). Si les incompatibilités HLA sont associées à une réplication plasmatique du BK-v ([16], [18]), leur impact sur le développement des Nx BK-v reste peu connu. L'une d'entre elles, Awadalla Y et al ([11]) a montré en 2004 que ces néphropathies sont associées à un taux élevé d'incompatibilité HLA. Ces auteurs ont donc suggéré qu'une diminution du nombre d'incompatibilité HLA réduirait le risque de développer ces néphropathies. Une remarque importante concernant cette étude est que cette association a été mise en évidence chez des patients avec Nx BK-v comparé à des patients sans Nx BK-v, sans préciser la présence d'une éventuelle virémie ou virurie chez les patients sans Nx BK-v. A l'inverse, des études plus récentes font état de la survenue de Nx BK-v également dans un contexte de bonne compatibilité HLA entre le donneur et le receveur ([17] ; [19]). Enfin, d'autres auteurs ont retrouvé chez des patients avec Nx BK-v une relation inverse entre un faible niveau d'incompatibilité HLA et une plus grande fréquence de pertes de greffon ([15]).

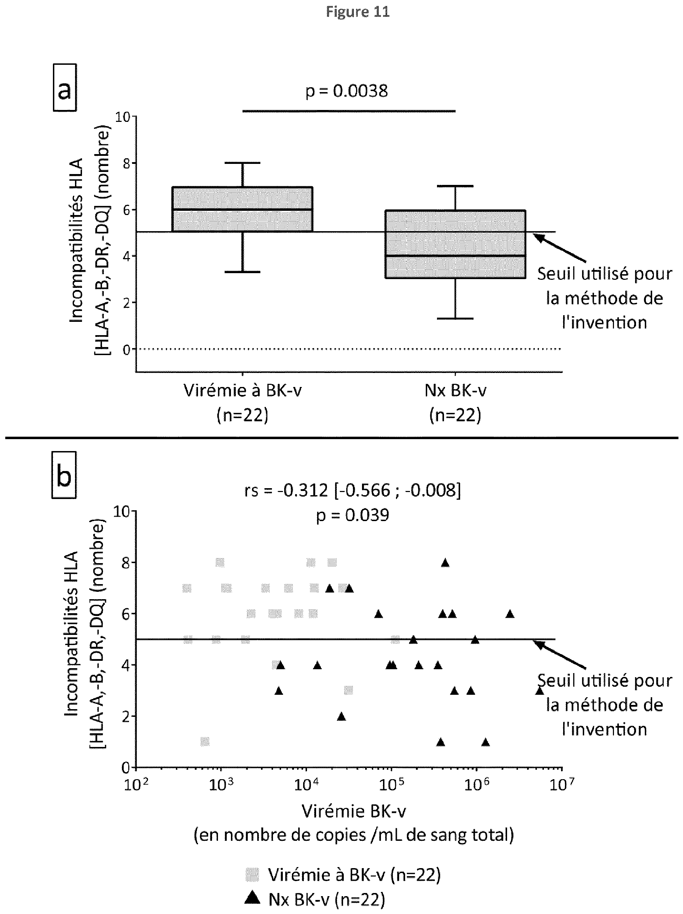

Basé sur les résultats ayant permis l'élaboration de la présente méthode, les inventeurs ont mis en évidence un nombre plus faible d'incompatibilité HLA chez les patients avec Nx BK-v. Les patients avec Nx BK-v présentaient un nombre total d'incompatibilité HLA [HLA-A, -B, -DR, -DQ] moindre par rapport aux patients avec virémie BK-v isolée (figure 11a). Par ailleurs, ce nombre d'incompatibilité HLA était négativement corrélé à la charge virale BK-v (figure 11b).

A partir de ces résultats, la valeur seuil de 5 incompatibilités dans les HLA-A, -B, -DR et -DQ a été intégré comme paramètre génétique dans le calcul de la présente méthode. Cette valeur seuil de 5 a été définie en fonction de l'étendue de la distribution non gaussienne du nombre d'incompatibilités HLA afin de prendre en compte jusqu'au 75$^{ème}$ percentile des valeurs d'incompatibilités HLA dans le groupe des patients avec virémie BK-v (figure 11a).

### *Compilation des résultats pour calculer l'index diagnostic*

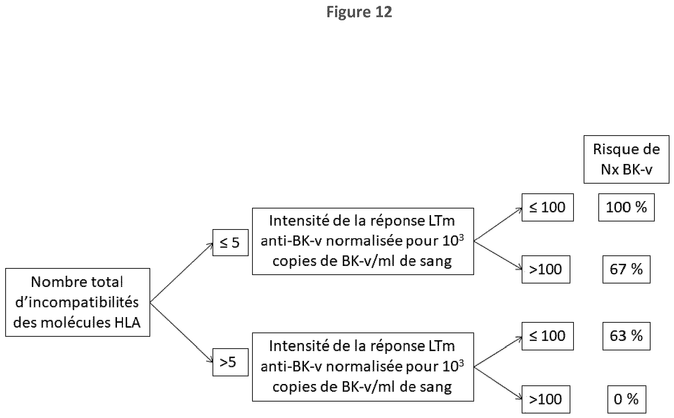

Les trois paramètres a), b) et c) décrits ci-dessus sont ensuite compilés pour créer un index de stratification du risque que le patient développe - ou non - une Nx BK-v. Ainsi, les présents inventeurs ne proposent pas d'utiliser les trois paramètres a), b) et c) isolément, mais de les combiner les uns aux autres afin de créer la méthode de l'invention et d'évaluer le risque de développer une Nx BK-v. La figure 12 représente l'ensemble des étapes décisionnelles menant à évaluer ce risque de Nx BK-v selon la méthode de l'invention.

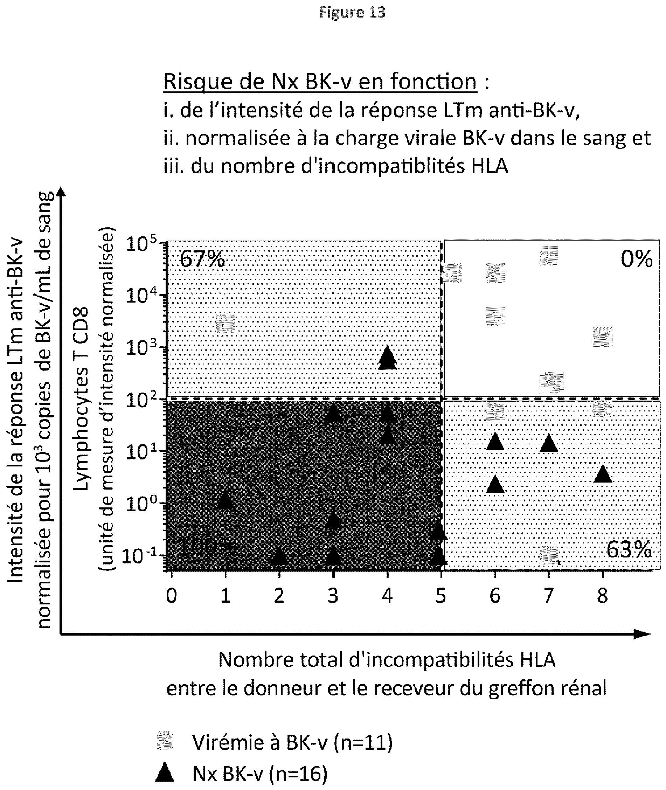

En effet, comme décrit dans les exemples ci-dessous et sur les figures 12 et 13, il existe des valeurs seuils, définies à partir des combinaisons des 3 paramètres utilisés dans la méthode de l'invention, qui permettent de distinguer les patients ayant un risque élevé de développer une Nx BK-v de ceux qui n'en développeront pas.

Basé sur la cohorte utilisée dans les exemples de la présente demande, ces seuils sont de :

- 5 pour le nombre d'incompatibilité HLA, et de
- $10^2$ unités de mesure d'intensité normalisée de la réponse LTm anti-BK-v.

Les présents inventeurs ont ainsi démontré qu'une intensité normalisée de la réponse LTm anti-BK-v inférieure ou égale à ($\leq$) $10^2$ associé à un nombre total d'incompatibilités HLA inférieur ou égal à ($\leq$) 5 traduit un risque élevé que le patient receveur de la greffe développe un jour une Nx BK-v à court, moyen ou long terme (figure 13 - cadran gris foncé).

A l'inverse, une intensité normalisée de la réponse LTm anti-BK-v strictement supérieure à (>) $10^2$ associé à un

nombre total d'incompatibilités HLA strictement supérieur à (>) 5 traduit un risque très faible que le patient receveur de la greffe développe une Nx BK-v à court, moyen ou long terme (figure 13 - cadran blanc).

En analyse transversale de la cohorte utilisée dans les exemples de la présente demande, 100% des patients ayant une intensité normalisée de la réponse LTm anti-BK-v $\leq 10^2$ et un nombre total d'incompatibilités HLA $\leq 5$ avaient une Nx BK-v (figure 13, cadran gris foncé). A l'inverse, aucun patient ayant une intensité normalisée de la réponse LTm anti-BK-v $> 10^2$ et un nombre total d'incompatibilités HLA > 5 n'avaient de Nx BK-v (figure 13, cadran blanc).

Ces seuils (5 pour le nombre d'incompatibilité HLA et $10^2$ pour les unités de mesure d'intensité normalisée de la réponse LTm anti-BK-v) ont été établis à partir du cas particulier de la cohorte étudiée pour élaborer la présente invention. Ces seuils pourront être amener à évoluer et/ou être affiner selon les résultats d'une cohorte plus conséquente suivie de manière longitudinale. Les présents inventeurs pourront plus généralement adapter la méthode de l'invention afin de tenir compte des spécificités d'une telle population. Notamment, pour obtenir l'index de l'invention, il sera possible aux présents inventeurs d'étudier les valeurs des trois paramètres a), b) c) mentionnés ci-dessus, de les combiner et d'identifier les seuils adaptés et les valeurs de normalisation adéquates en fonction des résultats et des méthodes graphiques obtenus à partir de l'analyse d'une telle population (méthode graphique sur le format des figures 12 et 13).

Dans le cas particulier de la cohorte étudiée, trois niveaux de risque ont été identifiés par les inventeurs sur la base de leurs analyses, réalisées sur plus de 27 patients transplantés rénaux (dont 11 patients avec virémie BK-v et 16 patients avec Nx BK-v) :

- Un <u>niveau de risque élevé de Nx BK-v</u> dans le cas d'une intensité normalisée de réponse LTm anti-BK-v $\leq 10^2$ et d'un nombre total d'incompatibilités HLA $\leq 5$ (figure 13 - cadran gris foncé). Ce niveau de risque est en faveur d'un risque accru de Nx BK-v. Une forte immunosuppression étant un facteur de risque de Nx BK-v, ce niveau de risque plaide pour un allégement important de l'immunosuppression et une surveillance rapprochée de la charge virale et de la fonction rénale.

- Un <u>niveau de risque intermédiaire de Nx BK-v</u> en présence d'une intensité normalisée de réponse LTm anti-BK-v $\leq 10^2$ et d'un nombre total d'incompatibilités HLA > 5 (figure 13 - cadran gris clair inférieur droit) ou en présence d'une intensité normalisée de réponse LTm anti-BK-v $> 10^2$ et d'un nombre total d'incompatibilités HLA $\leq 5$ (figure 13 - cadran gris clair supérieur gauche). Ce niveau de risque doit être considéré comme un niveau de risque intermédiaire de Nx BK-v.

- Un <u>niveau de risque faible de Nx BK-v</u> en présence d'une intensité normalisée de réponse LTm anti-BK-v $> 10^2$ et d'un nombre total d'incompatibilités HLA > 5 (figure 13 - cadran blanc). Ce niveau de risque est en faveur d'un faible risque de Nx BK-v. Dans cette situation, une surveillance de la charge virale et de la fonction rénale doit être préconisée. L'immunosuppression peut être maintenue ou légèrement allégée.

Dans le cas particulier de la cohorte étudiée, cet index a démontré les excellentes caractéristiques techniques suivantes :

- une valeur prédictive positive de 100 % (probabilité de Nx BK-v si le test est positif) lorsque le résultat de la méthode est un niveau de risque élevé de Nx BK-v,

- une valeur prédictive négative de 100 % (probabilité d'absence de Nx BK-v si le test est négatif) lorsque le résultat de la méthode est un niveau de risque faible de Nx BK-v,

- une sensibilité de plus de 60 % (probabilité de test positif en cas de Nx BK-v) lorsque le résultat de la méthode est un niveau de risque intermédiaire de Nx BK-v,

- Un taux nul de faux positif (un test positif étant défini par une intensité normalisée de réponse LTm anti-BK-v $\leq 10^2$ et un nombre total d'incompatibilités HLA $\leq 5$) dans le cas où la méthode indique un niveau de risque élevé de Nx BK-v.

Ainsi, l'index de l'invention dispose d'une valeur prédictive positive supérieure à celle classiquement décrite dans la littérature pour l'évaluation du risque de Nx BK-v en fonction de la charge virale sanguine du BK-v (100% *vs* 55%), ainsi que d'un taux nul de faux positif dans le cas où la méthode indique un niveau de risque élevé de Nx BK-v. Le tableau 1 illustre les performances des différents tests associés à la prédiction du risque de Nx BK-v (en fonction de la charge virale sanguine du BK-v vs la méthode de l'invention).

Tableau 1 : Performances des différents tests associés à la prédiction du risque de Nx BK-v (en fonction de la charge virale sanguine du BK-v vs la méthode de l'invention).

| Etude | Technique | Seuil de positivé | VPP (%) | VPN (%) |
|---|---|---|---|---|
| Présents inventeurs | Présente invention : Combinaison de 3 biomarqueurs | Unité de mesure d'intensité normalisée de réponse LTm anti-BK-v $\leq 10^2$ et Nombre d'incompatibilités HLA $\leq 5$ | 100 | 100 |
| Chung et al. [25] | PCR BK-v plasmatique | $> 10^4$ copies/mL | 54.5 | 100 |
| Godinho Pinto et al. [26] *(meta-analysis)* | PCR BK-v plasmatique | $> 4.2 \log_{10}$ | 50 | 100 |
| Godinho Pinto et al. [26] *(meta-analysis)* | PCR BK-v plasmatique | $\geq 3.7 \log_{10}$ | 29 | 100 |

La méthode de l'invention se caractérise donc de préférence en ce qu'un risque élevé de développer une néphropathie à BK virus (BK-v) est diagnostiqué si :

a) ramené à une charge virale de $10^3$ copies de virus BK-v par mL de sang,

b) l'intensité normalisée de la réponse LTm anti-BK-v CD4$^+$ ou CD8$^+$ est inférieure ou égale à 100 unités de mesure d'intensité, et

c) le nombre d'incompatibilité entre ledit patient et le donneur du rein greffé est inférieur ou égal à 5.

La méthode de l'invention se caractérise également de préférence en ce qu'un risque faible de développer une néphropathie à BK virus (BK-v) est diagnostiqué si :

a) ramené à une charge virale de $10^3$ copies de virus BK-v par mL de sang,
b) l'intensité normalisée de la réponse LTm anti-BK-v CD4$^+$ ou CD8$^+$ est strictement supérieure à 100 unités de mesure d'intensité, et
c) le nombre d'incompatibilité HLA entre ledit patient et le donneur de greffe est strictement supérieur à 5.

### Utilisations diagnostique et pronostique de l'index

N'étant pas invasive, la méthode de l'invention pourra être reproduite autant de fois que nécessaire chez les patients à risque (typiquement, les patients ayant subi une transplantation rénale). Sa fréquence pourra être personnalisée selon le profil de chaque patient, ou au vu des résultats des tests précédents. En outre, il sera possible de d'adapter la dose des traitements immunosuppresseurs de manière personnalisée, à la hausse ou à la baisse, à l'aide du résultat de cet index. La diminution du traitement sera prescrite uniquement pour les patients souffrant de Nx BK-v ou ceux dont le risque d'en développer est élevé. Les patients pour lesquels cette diminution serait sans bénéfice voire néfaste pour le greffon (typiquement les patients présentant une virémie isolée du virus BK-v sans atteinte du greffon rénal) seront identifiés et le traitement initialement prescrit pourra être maintenu.

➢ Il est recommandé de réaliser la méthode de l'invention pour chaque patient ayant subi une transplantation rénale, et ce à intervalle de temps régulier. Cette récurrence pourrait en effet permettre :

➢ D'aider au diagnostic de Nx BK-v

➢ De préciser individuellement le niveau de risque de développer une Nx BK-v afin de prévenir la survenue d'une telle complication.

➢ D'évaluer l'immunosuppression thérapeutique du patient, et, en particulier, de guider l'adaptation du traitement immunosuppresseur afin d'éviter tout défaut ou excès d'immunosuppression thérapeutique.

Dans un mode de réalisation préféré, la méthode de l'invention est réalisée en même temps que le dosage des traitements immunosuppresseurs à administrer audit patient. Un test concluant à un risque élevé de Nx BK-v serait en faveur d'une diminution du traitement immunosuppresseur afin de prévenir sa survenue.

Dans un mode de réalisation encore plus préféré, la méthode de l'invention est répétée plusieurs fois au cours du traitement post-transplantation, par exemple tous les 3 à 6 mois dans la population des patients à risque de développer une Nx BK-v (patients avec virémie et/ou ayant eu un traitement de rejet de greffe et/ou ayant une dégradation inexpliquée de la fonction du greffon rénal).

En plus de la réalisation systématique chez tous patients présentant une virémie BK-v, il est recommandé de réaliser la méthode de l'invention dans les situations suivantes :

    ◦ Lors de l'intensification du traitement immunosuppresseur dans le cadre du traitement curatif d'un rejet de greffe, afin d'évaluer le risque ultérieur de survenue de Nx BK-v,

    ◦ Lors de l'allègement de l'immunosuppression après un premier test positif, afin d'évaluer l'impact de cet allègement sur la réponse LTm anti-BK-v,

    ◦ En cas de dégradation inexpliquée de la fonction du greffon rénal afin d'évaluer le risque et/ou d'aider au diagnostic de Nx BK-v.

La méthode de l'invention pourrait être réalisée 2 à 3 mois après la modification du traitement immunosuppresseur. Le résultat de la méthode de l'invention pourrait permettre de définir chez un malade le moment de ré-augmentation du traitement immunosuppresseur, si une réponse immune se développe.

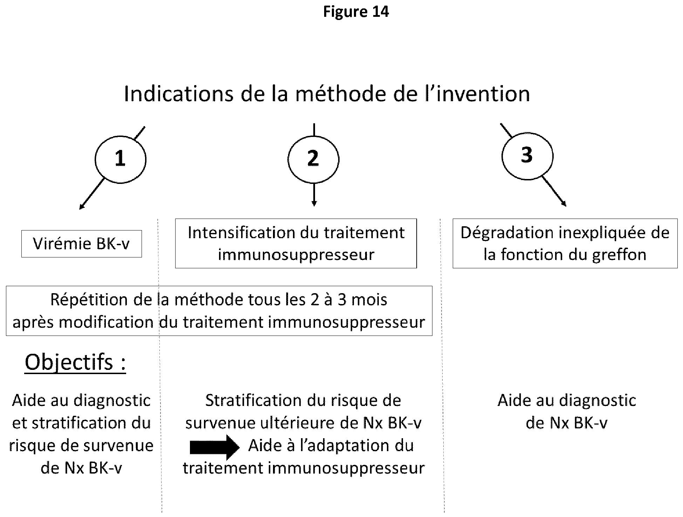

La figure 14 montre les différentes indications de la méthode de l'invention ainsi que le délai de répétition de ladite méthode.

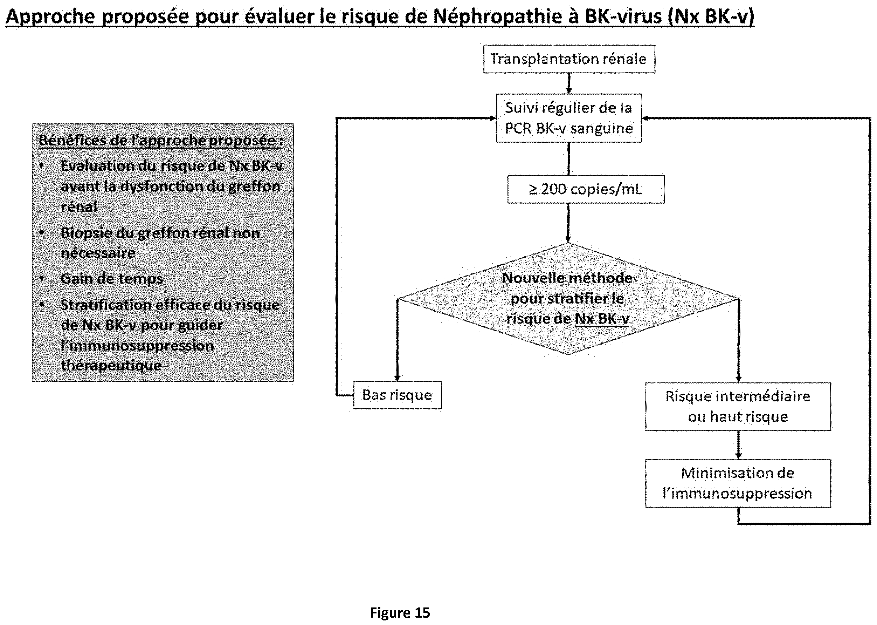

Ainsi, la figure 15 illustre l'approche proposée pour évaluer le risque de Nx BK-v. Cette nouvelle approche se base initialement sur le suivi longitudinal de la charge virale du BK-v dans le sang du patient, et ce dès le seuil de positivité de la charge virale BK-v (≥200 copies/mL de sang). A la différence de l'approche habituelle de la Nx BK-v (figure 3), cette nouvelle approche n'est pas conditionnée par la réalisation d'une biopsie du greffon rénal mais par l'utilisation de la méthode de l'invention. L'application de la présente méthode permet de stratifier le niveau de risque de Nx BK-v chez les patients transplantés rénaux avec réactivation BK-v sanguine. Sans que cela ne soit limitant, seuls les patients avec un risque de Nx BK-v évalué comme intermédiaire à élevé auront effectivement une minimisation du traitement immunosuppresseur. Les patients avec un risque faible de Nx BK-v n'auront pas de minimisation du traitement immunosuppresseur. Dans tous les cas, une surveillance régulière de la charge virale BK-v et la répétition de la dite-méthode seront également préconisées. La présente invention permet une évaluation du risque de Nx BK-v avant la dysfonction du greffon rénal. Elle permet un gain de temps et une stratification efficace du risque de Nx BK-v, permettant à terme de guider l'immunosuppression thérapeutique (figure 15).

La présente demande vise également une méthode *in vitro* d'évaluation de la réponse d'un patient greffé rénal à une réplication sanguine du BK-v après modification du traitement immunosuppresseur, caractérisée en ce que la méthode définie ci-dessus (index) est répétée à intervalle de temps régulier après la transplantation.

De préférence, la méthode de l'invention est répétée avant et après chaque modification de traitement.

De préférence, la méthode de l'invention est répétée tous les 3 à 6 mois environ, à une fréquence dépendant du niveau de la réactivation Bk-v plasmatique et/ou de la nécessité de modifier le traitement immunosuppresseur dudit patient.

Les traitements immunosuppresseurs couramment utilisés en transplantation rénale sont par exemple:

-    Au moment initial de la transplantation rénale, des traitements d'induction associant des corticostéroïdes à fortes doses et des anticorps polyclonaux anti-lymphocytaires (immunoglobulines anti-thymocytes humains, Thymoglobulines® ou Grafalon®) ou des anticorps monoclonaux anti-CD25 (Basiliximab, Simulect®) ;
-    En relais des traitements d'induction, des traitements d'entretien associant

        ◦ une corticothérapie à faible dose,
        ◦ un inhibiteur de la calcineurine (Tacrolimus, Prograf® ou Ciclosporine, Néoral®) et un inhibiteur de la synthèse des bases puriques (Mycophénolate mofétil, Cellcept®),
        ◦ ou encore d'autres molécules telles que des inhibiteurs de la voie mTor (Everolimus, Certican®) ou du CTLA4-Ig (Bélatacept, Nulojix®) [1].

Si la méthode de l'invention détecte un risque important de développer une Nx BK-v, la prise en charge thérapeutique doit aboutir à un allègement de l'immunosuppression, ou éventuellement à un changement de traitement.

## EP 3 807 635 B1

***Kit***

Selon un autre aspect, la présente invention concerne l'utilisation d'un kit contenant :

a) des réactifs permettant la réalisation d'une PCR quantitative du virus BK-v, et
b) des séquences peptidiques du BK-v ou des séquences peptidiques polyclonales de stimulation lymphocytaire,

pour mettre en oeuvre la méthode des revendications 1 à 12. Selon un autre aspect, la présente demande décrit un kit permettant la mise en œuvre sur sang total des méthodes décrites précédemment.

Ce kit contient par exemple:

a) des réactifs permettant la réalisation d'une PCR quantitative BK-v, et
b) des réactifs permettant la réalisation sur sang total de la méthode immunologique décrite ci-dessus.

De préférence, ledit kit contient des amorces spécifiques permettant d'amplifier des portions du génome du BK-v, et optionnellement les réactifs nécessaires à la réalisation d'une PCR quantitative avec extraction d'ADN (dNTP, enzyme, tampon, sonde et standard).

Ces amorces sont par exemple SEQ ID NO :1 (CATAGCATGCAAGGGCAGTG) et SEQ ID NO :2 (GAGCTG CCTGGGGAAATCTT) qui permettent d'amplifier une séquence de 281 nucléotides au sein du génome du virus BK-v.

Il est possible d'inclure des sondes permettant d'accroître la spécificité de la réaction de PCR. Une telle sonde a par exemple pour séquence SEQ ID NO:3 (TAGGCCATTCCTTGCAGTAC).

De préférence, ledit kit contient les réactifs nécessaires à la stimulation lymphocytaire, par exemple des tubes de culture cellulaire contenant (ou pas) des agents stimulants (par exemple des séquences peptidiques du BK-v ou des séquences peptidiques polyclonales de stimulation lymphocytaire de type *Staphylococcal Enterotoxin B (SEB)* ou Phytohaemagglutinin P (PHA), Phorbol 12-Myristate 13-Acetate (PMA) - Ionomycine), et optionnellement les réactifs nécessaires au marquage lymphocytaire (par exemple, un marqueur nucléaire de prolifération lymphocytaire de type Ki67 ou CFSE, une solution de lyse des globules rouges de type RBC Lysis Buffer et/ou de l'éthanol 70%).

De manière plus préférée, le kit contient :

a) des amorces spécifiques permettant d'amplifier des portions du génome du BK-v (telles que celles fournies dans les kits de détection du virus BK-V) et

b) des séquences peptidiques du BK-v et/ou des séquences peptidiques polyclonales de stimulation lymphocytaire, de préférence des mélanges de peptides chevauchants, tels que Peptivator®BKV VP1 ou Peptivator®BKV LT (peptides de 15-mer se chevauchant sur 11 acides aminés),
et, optionnellement,

c) les réactifs nécessaires à la réalisation d'une PCR quantitative avec extraction d'ADN ou

d) les réactifs nécessaires au marquage lymphocytaire,

tels que décrits ci-dessus.

De plus, le kit pourrait fournir les références d'un logiciel informatique permettant le calcul automatisé du nombre d'incompatibilités HLA et de l'index de risque de Nx BK-v.

Ce kit est utilisé pour mettre en œuvre les méthodes de l'invention dans des laboratoires d'analyses médicaux-hospitaliers et/ou privés, afin d'obtenir un index chiffré du risque de développer une Nx BK-v.

### Légende des figures

La **figure 1** décrit la fréquence de réactivation du BK-v après transplantation rénale en termes de virurie à BK-v, de virémie à BK-v isolée, de Nx BK-v et de dysfonction chronique/perte de greffons rénaux [4,5,6,7].

La **figure 2** décrit l'évolution à 3 ans du greffon rénal chez les patients avec ou sans Nx BK-v (a - fonction rénale évaluée par la mesure du débit de filtration glomérulaire (DFG) en ml/min/1.73m$^2$, test de Kruskal-Wallis et b - survie des greffons rénaux, Kaplan-Meier et log-rank test).

La **figure 3** représente l'approche actuelle de la Nx BK-v, basée sur l'évaluation de la charge virale sanguine du BK-v et sur la biopsie du greffon rénal.

La **figure 4** décrit la méthode de l'invention basée sur la combinaison de trois paramètres :

- i) un paramètre virologique évalué par la charge virale du BK-v dans le sang du patient,
- ii) un paramètre immunologique évalué par l'intensité de la réponse LTm anti-BK-v chez le patient, et
- iii) un paramètre génétique évalué par le nombre d'incompatibilités HLA dans le couple [donneur/receveur]

La **figure 5** montre les niveaux de charges virales BK-v dans le sang (a) et l'urine (b) des différents groupes de patients de la cohorte étudiée ici (test non paramétrique de Kruskal-Wallis). Le seuil utilisé dans la méthode de l'invention ($10^3$ copies/mL de BK-v dans le sang) a été défini à partir de ces résultats afin de permettre une évaluation du risque la plus précoce possible.

La **figure 6** - **partie 1** montre les différents tests de fonctionnalité lymphocytaire réalisés après stimulation par des peptides spécifiques du BK-v. La sécrétion cytokinique (1a) et la prolifération lymphocytaire (1b) ont été utilisées pour évaluer la réponse LTm anti-BK-v dans les différents groupes de patients de la cohorte étudiée ici (LT : lymphocytes T ; test non paramétrique de Kruskal-Wallis).

La **figure 6** - **partie 2** montre les différents tests de fonctionnalité lymphocytaire réalisés après stimulation par un mélange de peptides antiviraux (peptides couvrant les virus Cytomégalovirus, Epstein-Barr et Influenza). La sécrétion cytokinique (2a) et la prolifération lymphocytaire (2b) ont été utilisées pour évaluer la réponse lymphocytaire T mémoire antivirale globale dans les différents groupes de patients de la cohorte étudiée ici (pas de différences significatives).

La **figure 7** montre la corrélation négative entre l'intensité de la réponse LTm anti-BK-v et la charge virale BK-v (copies /mL de sang) (test de corrélation non paramétrique de Spearman).

La **figure 8/1** illustre le protocole d'évaluation de la réponse LTm anti-BK-v, via l'analyse de la prolifération des LTm anti-BK-v en réponse à un pool de peptides spécifique du BK-v (proportion de lymphocytes T CFSE^low). La proportion des LTm anti-BK-v ayant activement proliférés (proportion de LT CFSE^low) est mesurée en soustrayant la proportion de LT CFSE^low mesurée en absence de stimulation peptidique à la proportion de LT CFSE^low mesurée en présence des peptides spécifiques du BK-v.

La **figure 8/2** montre l'évaluation de l'intensité normalisée de la réponse LTm anti-BK-v.
L'intensité normalisée de la réponse LTm anti-BK-v est quantifiée en « unité de mesure d'intensité normalisée ». Une « unité de mesure d'intensité normalisée » est ici définie comme étant la proportion de LTm anti-BK-v CFSE^low, exprimée pour $10^6$ lymphocytes T totaux et normalisée pour $10^3$ copies de BK-v par mL de sang. Cette normalisation permet une comparaison intra et inter-individus [ici, comparaison inter-individus (virémie BK-v isolée vs Nx BK-v)].

La **figure 9** montre l'intensité normalisée de la réponse LTm anti-BK-v CD8⁺ chez des patients souffrant de virémie à BK-v isolée (n=11) ou de Nx BK-v (n=16). Le seuil utilisé dans la méthode de l'invention ($10^2$ unités de mesure d'intensité normalisée) a été défini à partir de ces résultats afin de prendre en considération jusqu'au 90^ème percentile des valeurs d'intensité de réponse mis en évidence chez les patients avec Nx BK-v.

La **figure 10** montre la comparaison de l'intensité normalisée de la réponse LTm anti-BK-v dans les lymphocytes de type CD4 et CD8 chez des patients avec virémie BK-v isolée (n=11) et des patients avec Nx BK-v (n=16).

La **figure 11a** montre un nombre plus faible d'incompatibilités HLA [HLA-A, -B, -DR, -DQ] entre le donneur et le receveur de greffe chez les patients avec Nx BK-v par rapport aux patients avec virémie BK-v isolée (Mann-Whitney test). Le seuil utilisé dans la méthode de l'invention (5 incompatibilités HLA [HLA-A, -B, -DR, -DQ] entre le donneur et le receveur de greffe) a été défini à partir de ces résultats afin de prendre en compte jusqu'au 75^ème percentile des valeurs d'incompatibilités HLA chez les patients avec virémie BK-v isolée.

La **figure 11b** montre la corrélation négative entre le nombre d'incompatibilités HLA et la virémie BK-v (en nombre de copies /mL de sang) (test de corrélation non paramétrique de Spearman).

La **figure 12** est un arbre de stratification du risque de Nx BK-v selon les 3 paramètres définis ci-dessus.

La **figure 13** décrit le niveau de risque de développer une Nx BK-v après transplantation rénale en cas de virémie BK-v en fonction des trois paramètres suivants :

- i. l'intensité de la réponse LTm anti-BK-v normalisée à,
- ii. la charge virale BK-v sanguine ($10^3$ copies de BK-v par mL de sang), et
- iii. le nombre d'incompatibilités HLA entre le donneur et le receveur.

La **figure 14** montre les différentes indications de la méthode de l'invention ainsi que le délai de répétition de ladite méthode.

La **figure 15** montre l'approche proposée ici pour évaluer le risque de Nx BK-v, basée sur l'évaluation de la charge virale sanguine de BK-v et la méthode de l'invention.

## Exemples

**Matériel et méthodes** :

Afin de caractériser la réponse cellulaire spécifique du BK-v en post-transplantation rénale, les présents inventeurs ont conduit une étude observationnelle incluant 94 patients transplantés rénaux. Les patients inclus sont tous issus du service de Néphrologie de l'hôpital Bicêtre (Hôpitaux Universitaires Paris Sud). Au total, ce centre réalise plus de 130 greffes rénales par an, avec une file active de plus 2000 patients suivis avec un greffon rénal fonctionnel. Le CPP Ile de France a été consulté et n'a pas retrouvé d'obstacle éthique à la réalisation de cette étude. Une lettre d'information a été donnée à l'ensemble des patients, leur consentement ayant été recueilli par écrit.

Cette étude a débuté en Novembre 2014 avec une durée d'inclusion de 36 mois (inclusions finies au 01 Novembre 2017). Les critères d'inclusion ont été les suivants :

➢ patients âgés de plus de 18 ans,
➢ pas d'autre transplantation d'organe hormis celle rénale,
➢ pas d'infection VIH/VHB/VHC chronique active.

Quatre groupes de réactivation du virus BK-v ont été définis chez ces patients transplantés rénaux selon les critères ci-dessous :

➢ patients sans réactivation BK-v :

　○ charges virales BK-v plasmatique et urinaire indétectables pendant au moins 12 mois

➢ patients avec virurie BK-v :

　○ virurie BK-v positive (> 200 copies/ml) et
　○ virémie BK-v indétectable pendant au moins 12 mois

➢ patients avec virémie BK-v isolée :

　○ virémie BK-v positive (> 200 copies/ml) depuis au moins 6 mois et
　○ absence de diagnostic histologique de Nx BK-v à la biopsie de greffon rénal

➢ patients avec Nx BK-v :

　○ diagnostic de Nx BK-v sur la biopsie de greffon rénal
　○ avec virémie BK-v positive (> 200 copies/ml).

Afin de caractériser les LTm anti-BK-v dans les différents groupes de patients de cette cohorte, la présente étude a comporté un versant clinique (recueil exhaustif de données cliniques et biologiques sur un suivi prospectif de 36 mois) ainsi qu'un versant immunologique (étude des cellules mononuclées du sang total (PBMC) sur prélèvement de sang périphérique).

Concernant la partie clinique de l'étude, les données clinico-biologiques suivantes ont été recueillies dans le cadre d'un suivi longitudinal de 36 mois :

➢ Age, sexe, maladie rénale initiale, méthode et ancienneté de dialyse du receveur
➢ Caractéristiques de la transplantation rénale :

○ Ancienneté de la transplantation rénale
○ Type de donneur
○ Rang de transplantation (1ère greffe ou 2ème/3ème/... greffe),

➢ Rejet de greffe d'organe
➢ Réactivation du virus BK-v :

○ Charges virales BK-v sanguines et urinaires (exprimés en copies/mL)
○ Durée de réplication (exprimée en mois)

➢ Estimation du Débit de Filtration Glomérulaire selon la formule MDRD :

○ A 1 mois et 6 mois post-greffe
○ Au diagnostic de Nx BK-v ou à 12 mois post-greffe
○ En fin de suivi

➢ Traitement immunosuppresseur :

○ Traitement d'induction et traitement d'entretien
○ Taux résiduel d'immunosuppresseurs

■ au diagnostic de Nx BK-v ou à 12 mois post-transplantation rénale

○ Modification du traitement immunosuppresseur dans les suites du diagnostic de Nx BK-v.

Le **tableau 2** montre l'absence de différence en termes d'immunosuppression thérapeutique (nature et intensité du traitement immunosuppresseur) dans les différents groupes de patients transplantés rénaux (*NS* = non significatif).

Tableau 2: Traitement immunosuppresseur utilisé dans les différents groupes de patient transplantés rénaux (NS = non significatif)

| Traitement immunosuppresseur | Sans réactivation à BK-v | Virurie BK-v | Virémie BK-v | Nx BK-v | p |
|---|---|---|---|---|---|
| n | 25 | 25 | 22 | 22 | |
| TRAITEMENT D'INDUCTION (n, %) | 23 (92) | 22 (88) | 22 (100) | 21 (95.5) | *NS* |
| Anticorps déplétants polyclonaux (n, %) | 11 (47.8) | 6 (27.3) | 11 (50) | 12 (57.1) | *NS* |
| Anticorps monoclonal anti-CD25 (n, %) | 12 (52.2) | 16 (72.7) | 11 (50) | 9 (42.9) | |
| TRAITEMENT D'ENTRETIEN avant le diagnostic de néphropathie à BKv | | | | | |
| Tacrolimus (n, %) | 18 (72) | 19 (76) | 15 (68.2) | 17 (77.3) | *NS* |
| Taux résiduel de Tacrolimus (ng/ml, médiane avec interquartile) | 9.1 [8.1-11.4] | 9.1 [8.4-10.4] | 9.1 [7.7-11.11 | 8.6 [6.6-10.11 | *NS* |
| Antimétabolites (n, %) | 23 (92) | 23(92) | 15 (68.2) | 18 (81.8) | *NS* |
| Glucocorticoides (n. %) | 25 (100) | 25 (100) | 22 (100) | 22 (100) | *NS* |
| TRAITEMENT DE REJET DE GREFFE avant le diagnostic de néphropathie à BKv | | | | | |
| Bolus de glucocorticoides intra-veineux (n, %) | 5 (20) | 5 (20) | 8 (36.4) | 10 (45.5) | *NS* |
| Anticorps déplétants polyclonaux (n, %) | 1 (4) | 1 (4) | 3 (13.6) | 1 (4.6) | *NS* |
| Echanges plasmatiques (n, %) | 1 (4) | 4 (16) | 5 (22.7) | 4 (18.2) | *NS* |
| RITUXIMAB (n, %) | 2 (8) | 2 (8) | 4 (18.2) | 2 (9.1) | *NS* |

Concernant la partie immunologique de l'étude, un prélèvement sanguin unique de 15 ml de sang total (3 tubes héparinates de lithium de 5 ml) a été réalisé à l'inclusion des patients. Ce prélèvement est effectué lors des bilans biologiques réalisés pour le suivi habituel des patients. Dans les groupes avec virémie à BK-v isolée et Nx BK-v, un suivi longitudinal a été également réalisé, avec la réalisation d'un nouveau prélèvement à 12 et 24 mois post-inclusion afin d'évaluer l'évolution de la réponse LTm anti-BK-v. A partir de 15 ml de sang total, 8 à 10 millions de PBMC ont été isolées en moyenne. Ceci témoigne de la lymphopénie relative des patients (entre 0,5 et 0,6 millions de PBMC/ml de sang).

La réponse LTm anti-BK-v a été caractérisée dans les différents groupes de patients. Une activation spécifique des LTm anti-BK-v a été réalisée par la mise en culture des PBMC avec un pool de peptides chevauchants recouvrant les protéines VP1 et LT-Ag du BK-v (PepTivator BKV, société Miltenyi® ou équivalents). Ces pools de peptides activent les lymphocytes T CD4 et CD8 et couvrent les 2 cycles de réplication du BK-v (LT-Ag pour la phase précoce et VP1 pour la phase tardive de la réplication du virus BK-v). Après activation par les peptides spécifiques du BK-v, la polyfonctionnalité lymphocytaire des LTm anti-BK-v a été évaluée par cytométrie de flux en termes de :

➢ Sécrétions cytokiniques :

◦ incubation sur la nuit de 4 millions de PBMC (800.000 PBMC/condition) en présence de peptides spécifiques du BK-v et de Bréfeldine A (inhibiteur de sécrétion cytokinique) puis
◦ étude des capacités de synthèse d'IL2, d'IFN-$\gamma$ et de TNF-$\alpha$.

➢ Prolifération lymphocytaire :

◦ mise en culture de 4 millions de PBMC marquées au CFSE (500.000 PBMC/condition) pendant 5 jours en présence de peptides spécifiques du BK-v puis
◦ étude des capacités de prolifération par visualisation de la dilution du CFSE.

Toutes les acquisitions en cytométrie de flux ont été faites sur un cytomètre 18 couleurs (BD LSRFortessa™ ou équivalent) et les analyses ont été faites sur le logiciel d'analyse spécifique FlowJo.

Méthodologie statistique utilisée :

Les variables continues sont exprimées en médiane avec écart interquartiles [25-75] devant l'absence de distribution normale des données et des petits effectifs (n<30). Ces variables ont été comparées en utilisant des tests non paramétriques de Mann-Whitney ou de Kruskal-Wallis selon la comparaison de 2 ou de plusieurs distributions. Les corrélations ont été testées en utilisant le test de corrélation non-paramétrique de Spearman.

Les variables catégorielles sont exprimées en pourcentages. Le chi-square test a été utilisé pour comparer les proportions entre les différents groupes. Les fonctions de survie ont été faites selon l'estimateur de Kaplan-Meier, avec le test de comparaison de Logrank.

Le seuil de signification statistique a été établi pour une valeur de p < 0,05. L'analyse statistique a été réalisée avec le logiciel GraphPad Prism.

Elaboration de la méthode de l'invention :

a) Mesure de la prolifération des lymphocytes T mémoires spécifiques du BK-v

Ce test de fonctionnalité lymphocytaire basé sur l'évaluation des capacités de prolifération des LTm anti-BK-v après activation par des peptides BK-v a été spécifiquement développé par les présents inventeurs.

La **figure 8/1** illustre le protocole d'identification des LTm anti-BK-v ayant proliférés, en réponse à une stimulation spécifique par les peptides du virus BK-v, à partir du sang total des patients transplantés rénaux.

Les PBMC, comprenant les LTm anti-BK-v ont été obtenues à partir d'un échantillon sanguin de 15 mL de sang total pour chaque patient. Ce prélèvement sanguin est de type veineux périphérique, réalisé avec 3 tubes Héparinate de Lithium (tubes à bouchons verts) de 5mL (Figure 8/1a).

Le test de prolifération des LTm anti-BK-v nécessite environ 5 à 6 millions de PBMC, le prélèvement sanguin en fournissant 8 à 10 millions.

Les PBMC ont été isolées par gradient de FICOLL (PAN Biotech® - référence P04-60505 ou équivalent). A partir de 15 ml de sang total, les présents inventeurs isolent en moyenne 8 à 10 millions de PBMC (Figure 8/1b).

Les PBMC ont été mises en culture pendant 5 jours avec 2 pools de peptides du BK-v à la concentration finale de 1$\mu$g/mL/peptide. Les pools de peptides sont commercialisés par la société Myltenyi Biotec (Peptivator® BKV VP1 - référence : 130-097-272 et Peptivator® BKV LT - référence : 130-096-504) ou équivalents (Figure 8/1c). Les séquences peptidiques de LT-Ag et de VP1 utilisées sont des peptides immuno-dominants largement reconnus dans la littérature scientifique comme capables d'activer les LTm anti-BK-v. La plus forte des deux réponses a été prise en compte pour l'identification des LTm anti-BK-v fonctionnels.

La prolifération des lymphocytes T a été mesurée par l'ajout, le jour de la mise en culture, d'un marqueur de prolifération lymphocytaire (CellTrace CFSE Thermo Fisher Scientific® - référence : C34554). D'autres équivalents de marqueur de prolifération lymphocytaire peuvent également être utilisés. Au $5^{ème}$ jour de culture cellulaire, les PBMC ont été marquées par les anticorps suivants [anticorps anti-CD3 BD® (référence : 564712), CD4 Myltenyi® (référence : 130-096-900) et CD8 Myltenyi® (référence : 130-096-561) ou équivalents] et analysées par cytométrie de flux (cytomètre BD LSRFortessa™ ou équivalent) (Figure 8/1d). Les LTm spécifiques du BK-v CD4 et CD8 sont ainsi identifiés et dénombrés sur la base du niveau de marquage CFSE (lymphocytes T CFSE$^{low}$). Comme expliqué plus haut, la proportion des LTm anti-BK-v ayant activement proliférés (proportion de LT CFSE$^{low}$) est mesurée en soustrayant la proportion de LT CFSE$^{low}$ mesurée en absence de stimulation peptidique à la proportion de LT CFSE$^{low}$ mesurée en présence des peptides spécifiques du BK-v (Figures 8/1e et 8/2a).

La proportion des LTm anti-BK-v fonctionnels est ensuite exprimée pour $10^6$ lymphocytes T totaux puis normalisée pour $10^3$ copies de BK-v par mL de sang. La **figure 8/2** illustre cette normalisation de la proportion des LTm anti-BK-v fonctionnels afin de permettre une évaluation de l'intensité normalisée de la réponse LTm anti-BK-v. L'évaluation de l'intensité de cette réponse permet une comparaison intra et inter-individus.

b) Mesure de la charge virale

La mesure de la charge virale du BK-v a été effectuée via une trousse commerciale spécifique (BKV R-GENE® - référence 69-013B de BioMérieux® ou équivalent). L'échantillon biologique utilisé peut être un échantillon de sang total ou de plasma du dit patient.

c) Mesure du nombre d'incompatibilité HLA entre donneur et receveur

Tous les typages HLA dans le cadre de la transplantation sont réalisés par le laboratoire HLA de l'hôpital St Louis (qui centralise ces examens en Ile de France). Le typage HLA du receveur est réalisé actuellement par techniques de séquençage allélique de nouvelle génération (séquençage allélique des molécules HLA A, B, C, DRB1, DQB1, DQA1 et DPB1 - appareil MiSeq de la société Illumina® ou équivalent). Le typage HLA du donneur est réalisé en urgence, au moment de la transplantation rénale, à l'aide d'une méthode « Sequence Specific Primers method » (Linkage Biosciences, Thermofischer®) utilisant des trousses de type SABR (Single Antigen Bead Resolution) ou équivalent.

Il est important de rappeler que les typages HLA du donneur et du receveur sont réalisés selon les recommandations internationales de manière systématique pour l'ensemble des patients transplantés rénaux au moment de la greffe afin de décider de la transplantation rénale ou non. Les résultats sont transmis aux équipes médicales par l'intermédiaire de l'Agence de la Biomédecine.

**Résultats** :

Quatre-vingt-quatorze patients transplantés rénaux ont été inclus et répartis en 4 groupes selon leur niveau de réactivation du virus BK-v :

➤ Patients sans réactivation BK-v (n=25) :

◦ charges virales BK-v plasmatique et urinaire < 200 copies/mL

➤ Patients avec virurie BK-v (n=25) :

◦ charge virale BK-v médiane urinaire de $1,9.10^4$ [$3,9.10^3$ - $1,8.10^5$] copies/mL
◦ charge virale BK-v plasmatique < 200 copies/mL
◦ délai médian de réactivation de 21,8 [14,6 - 34,7] mois.

➤ Patients avec virémie BK-v isolée (n=22) :

◦ charge virale BK-v plasmatique de $4,2.10^3$ [$1,1.10^3$ - $1,2.10^4$] copies/mL
◦ charge virale BK-v urinaire de $1,1.10^8$ [$6,2.10^6$ - $3,8.10^8$] copies/mL
◦ délai médian de réactivation respectif de 10,3 [5,6 - 21,1] et 13,8 mois [9,3 - 27,1]
◦ absence de diagnostic histologique de Nx BK-v à la biopsie de greffon rénal

➢ Patients avec Nx BK-v (n=22):

  ◦ diagnostic histologique de Nx BK-v sur la biopsie de greffon rénal
  ◦ charge virale BK-v plasmatique de $2,8.10^5$ [$3.10^4$ - $6,3.10^5$] copies/mL
  ◦ charge virale BK-v urinaire de $1.10^9$ [$5,4.10^7$ -$1,8.10^9$] copies/mL
  ◦ délai médian de réactivation respectif de 12,6 [5,2 - 24,7] et 13,8 mois [4 - 23,3].

La figure 5 montre les différents niveaux de charges virales sanguines et urinaires du BK- v dans les différents groupes de patients en fonction du niveau de réactivation BK-v. En accord avec l'état de l'art antérieur, il est important de noter que si une charge virale plasmatique du BK-v > $10^5$ peut être associée avec une Nx BK-v (figure 5a), ce paramètre est tardif car déjà associé à des lésions du parenchyme rénal et à une dysfonction chronique sévère du greffon au moment du diagnostic de Nx BK-v (figure 2a). Par ailleurs, devant l'absence de différence entre les charges virales urinaires du BK-v chez les patients avec virémie BK-v isolée et Nx BK-v, ce paramètre n'a pas été considéré dans l'élaboration de la méthode de l'invention.

Sur les données clinico-biologiques obtenues à ce jour, le délai moyen de greffe de l'ensemble de la cohorte étudiée était de 3,5 ans. Le délai médian de survenue de Nx BK-v était de 12 mois après transplantation rénale.

Du fait d'une gestion standardisée monocentrique de l'immunosuppression, les groupes étaient comparables en termes d'immunosuppression (nature et intensité du traitement immunosuppresseur - cf. tableau 2).

Une dysfonction chronique sévère du greffon rénal était retrouvée dès le diagnostic de Nx BK-v (p<0.0001, figure 2a). L'évolution sur le plan rénal était également défavorable avec un taux plus élevé de perte de greffon chez les patients avec Nx BK-v comparé aux patients sans Nx BK-v (p<0.0001, figure 2b).

Une fonctionnalité altérée des LTm anti-BK-v a été mise en évidence chez les patients avec Nx BK-v en post-transplantation rénale. Le groupe de patients avec Nx BK-v présentait une réponse LTm anti-BK-v plus faible :

- en terme de capacité de sécrétion cytokinique : capacité de sécrétion d'Interféron $\gamma$ diminuée dans le groupe Nx BK-v par rapport au groupe avec virurie BK-v (figure 6/1a).
- en terme de capacité de prolifération lymphocytaire : capacité de prolifération diminuée dans le groupe Nx BK-v par rapport aux 3 autres groupes (patients sans réactivation à BK-v, avec virurie BK-v et virémie BK-v isolée) (figure 6/1b).

Cette altération de la réponse lymphocytaire était spécifique aux LTm anti-BK-v. Les patients avec Nx-BK v possédaient une préservation des réponses anti-virales après stimulation par un pool de peptides couvrant les virus Cytomégalovirus, Epstein-Barr et Influenza (CEF peptide pool de Axxora®, référence PT-PA-CEF-002 ou équivalent - figure 6/2a-b).

L'intensité de la réponse proliférative LTm anti-BK-v était négativement corrélée à la charge virale BK-v dans le sang (figure 7). Après normalisation de l'intensité de la réponse à $10^3$ copies de BK-v/mL de sang, cette intensité normalisée de la réponse anti-BK-v était significativement plus faible chez les patients avec Nx BK-v par rapport aux patients avec virémie BK-v isolée (figure 9). Ces données suggèrent une altération de la polyfonctionnalité lymphocytaire des LTm anti-BK-v, faisant évoquer un état d'épuisement de ces lymphocytes.

La prolifération lymphocytaire est la méthode d'évaluation de la fonctionnalité lymphocytaire la plus discriminante. Par ailleurs, ce test permet d'évaluer spécifiquement les lymphocytes T centraux mémoires et souches mémoires. Or, comme expliqué précédemment, l'importance de ces sous-populations lymphocytaires au sein des lymphocytes T mémoires spécifiques d'un agent pathogène détermine leurs capacités d'auto-renouvellement, de prolifération, de signaux d'aide et de différentiation en effecteurs. L'évaluation spécifique de ces lymphocytes centraux mémoires et souches mémoires permet une évaluation plus précise des capacités de protection immunologique contre le virus BK-v. L'évaluation des capacités de sécrétions cytokiniques permet d'étudier surtout des sous-populations lymphocytaires mémoires plus « terminales », de type lymphocytes T effecteurs mémoires ou effecteurs terminaux. Ces sous-populations lymphocytaires très différenciées sont peu, voire non dotées de capacités d'auto-renouvellement, de prolifération et de différentiation ([13] ; [14]). Cette méthode d'évaluation a donc été privilégiée.

Par ailleurs, comme mentionné plus haut, un nombre plus faible d'incompatibilité HLA a été mis en évidence chez les patients avec Nx BK-v. Les patients avec Nx BK-v présentaient un nombre total d'incompatibilité HLA [HLA-A, -B, -DR et -DQ] moindre par rapport aux patients avec virémie à BK-v isolée (figure 11a). Par ailleurs, ce nombre d'incompatibilité était négativement corrélé à la charge virale BK-v (figure 11b).

A partir des données obtenues, un index de stratification du risque de développer une Nx BK-v a été développé.

Chez les patients avec Nx BK-v, il a été possible de mettre en évidence une association entre :

- une diminution de l'intensité normalisée de la réponse LTm anti-BK-v (intensité de réponse normalisée pour $10^3$ copies/ml de sang de virus BK-v) et

**EP 3 807 635 B1**

- un faible nombre d'incompatibilités des molécules HLA entre le donneur et le receveur.

Ainsi, en analyse transversale sur la présente cohorte, 100% des patients ayant une intensité normalisée de réponse LTm anti-BK-v $\leq 10^2$ et un nombre total d'incompatibilités HLA $\leq 5$ avaient une Nx BK-v (figure 13, cadran gris foncé). A l'inverse, aucun patient ayant une intensité normalisée de réponse LTm anti-BK-v $> 10^2$ et un nombre total d'incompatibilités HLA $> 5$ n'avaient de Nx BK-v (figure 13, cadran blanc).

**Références bibliographiques**

[1] Kidney Disease: Improving Global Outcomes (KDIGO) Transplant Work Group. KDIGO clinical practice guideline for the care of kidney transplant recipients. Am J Transplant. 2009;9:S1-155.

[2] Agence de la Biomédecine. Rapport Annuel REIN (Réseau Epidémiologie et Information en Néphrologie) 2015. Disponible sur: https://www.agence-biomedecine.fr

[3] Maillart E, Taoufik Y, Gasnault J, Stankoff B. Leucoencéphalopathie multifocale progressive. EMC Neurol. 2017.

[4] Dalianis T, Hirsch HH. Human polyomaviruses in disease and cancer. Virology. 2013;437:63-72.

[5] Hirsch H, Vincenti F, Friman S, Tuncer M, Citterio F, Wiecek A, et al. Polyomavirus BK replication in de novo kidney transplant patients receiving tacrolimus or cyclosporine: a prospective, randomized, multicenter study. Am J Transplant. 2013;13:136-45.

[6] Hirsch H, Knowles W, Dickenmann M, Passweg J, Klimkait T, Mihatsch MJ, et al. Prospective study of polyomavirus type BK replication and nephropathy in renal-transplant recipients. N Engl J Med. 2002;347:488-96.

[7] Hirsch H, Babel N, Comoli P, Friman V, Ginevri F, Jardine A, et al. European perspective on human polyomavirus infection, replication and disease in solid organ transplantation. Clin Microbiol Infect. 2014;20:74-88.

[8] Dekeyser M, François H, Beaudreuil S, Durrbach A. Polyomavirus-Specific Cellular Immunity: From BK-Virus-Specific Cellular Immunity to BK-Virus-Associated Nephropathy? Front Immunol. 2015;6:307.

[9] Sharma R., Tzetzo S., Patel S., Zachariah Mareena, Sharma S., Melendy T. BK virus in Kidney Transplant: current Concepts, Recent Advances and Future Directions, Experimental and Clinical Transplantation (2016): 4:377-384

[10] Limaye AP, Jerome KR, Kuhr CS, Ferrenberg J, Huang ML, Davis CL, Corey L, Marsh CL. Quantitation of BK virus load in serum for the diagnosis of BK virus associated nephropathy in renal transplant recipients. J. Infect. Dis. 2001

[11] Awadalla Y., Randhawa P., Ruppert K., Zeevi A., Duquesnoy RJ. HLA Mismatching Increases the Risk of BK Virus Nephropathy in Renal Transplant Recipients. Am. J. Transplant 2004

[12] Sester M, Leboeuf C, Schmidt T & Hirsch H. The 'ABC' of Virus-Specific T Cell Immunity in Solid Organ Transplantation. Am. J. Transplant. (2016):16 :1697-1706.

[13] Restifo, Nicholas P., et Luca Gattinoni. « Lineage Relationship of Effector and Memory T Cells ». Current Opinion in Immunology (2013) 25 (5):556-63.

[14] Egli, A., Humar, A. & Kumar, D. State-of-the-art monitoring of cytomegalovirus-specific cell-mediated immunity after organ transplant: a primer for the clinician. Clin. Infect. Dis. (2012) 55, 1678-1689.

[15] Drachenberg, Cinthia B., John C. Papadimitriou, Dean Mann, Hans H. Hirsch, Ravinder Wali, et Emilio Ramos. 2005. « Negative Impact of Human Leukocyte Antigen Matching in the Outcome of Polyomavirus Nephropathy ». Transplantation 80 (2): 276-78.

[16] Hässig, A., M. Roos, A. Etter, W. Bossart, N. Müller, M. Schiesser, R. P. Wüthrich, et T. Fehr. 2014. « Association of BK Viremia with Human Leukocyte Antigen Mismatches and Acute Rejection, but Not with Type of Calcineurin Inhibitor ». Transplant Infectious Disease 16 (1): 44-54.

[17] Helanterä, Ilkka, Kaija Salmela, Lauri Kyllönen, Anne Räisänen-Sokolowski, Eeva Auvinen, Laura Mannonen, Petri Koskinen, et Irmeli Lautenschlager. 2012. « BK Virus Viremia in a Well-HLA-Matched Kidney Transplant Population Mainly on Low-Dose Cyclosporine-Based Immunosuppression ». Clinical Transplantation 26 (6): E596-601.

[18] Thangaraju, Sobhana, Jagbir Gill, Allissa Wright, Jianghu Dong, Caren Rose, et John Gill. 2016. « Risk Factors for BK Polyoma Virus Treatment and Association of Treatment With Kidney Transplant Failure: Insights From a Paired Kidney Analysis ». Transplantation 100 (4): 854-61.

[19] Ramos E, Drachenberg CB, Papadimitriou JC, et al. Clinical course of polyoma virus nephropathy in 67 renal transplant patients. J Am Soc Nephrol 2002; 13: 2145.

[20] Batal I. et al, Measurments of Global Cell-Mediated Immunity in Renal Transplant Recipients With BK virus reactivation. Am. J. Clin. Pathol. 2008; 129:857-591

[21] Comoli P. et al, Polyomavirus-associated nephropathy : update on BK virus-specific immunity. Transpl. Infect. Dis. 2006:8:86-94

[22] Schachtner T. et al, BY virus-Specific Immunity Kinetics: A predictor of Recovery From polyomavirus BK-associated nephropathy. Am. Journal. Of Transplantation, 2011; 11:2443-2452

[23] Schachtner T. et al, The Loss of BKV-specific immunity from Pretransplantation to Posttransplantation identifies Kidney Transplant Recipients at Increased Risk of BKV Replication. Am. Journal. Of Transplantation, 2015; 15:2159-2169

[24] Johnston, O. et al. Treatment of polyomavirus infection in kidney transplant recipients: a systematic review. Transplantation 89, 1057-1070 (2010).

[25] Chung, B. H. et al. Clinical usefulness of BK virus plasma quantitative PCR to prevent BK virus associated nephropathy. Transpl. Int. 25, 687-695 (2012).

[26] Godinho Pinto, G., Poloni, J., Rotta, L., R. Razonable, R. & Pasqualotto, A. Screening for BK virus nephropathy in kidney transplant recipients: comparison of diagnostic tests. J. Bras. Nefrol. 38, (2016)

[27] Comoli, P. et al. Immunity to Polyomavirus BK Infection: Immune Monitoring to Regulate the Balance between Risk of BKV Nephropathy and Induction of Alloimmunity. Clin. Dev. Immunol. 2013, 256923 (2013).

[28] Agence de la Biomédecine. Le rapport médical et scientifique du prélèvement et de la greffe en France en 2016. (2016).

[29] Suleiman B., Saleh S., Hassan W., Wang K., Maibam A., Karim A., Hines A., Mei X., El-Husseini A., Yaseen M., Cornea V., Castellanos A., Gedaly R., Waid T. HLA Mismatching and cPRA Do Not Affect BK Viremia and Nephropathy Risk in Kidney Transplantation: A Retrospective Analysis. 2018 American Transplant Congress.

[30] Lanot A., Bouvier N., Chatelet V., Dina J., Béchade C., Ficheux M., Henri P., Lobbedez T., Hurault de Ligny B. Infections à BK virus en transplantation rénale. Nephrologie & therapeutique, vol.12, N°2, 2016

**Revendications**

1. Méthode pour évaluer le risque de développer une néphropathie à BK virus (BK-v) chez un patient ayant subi une transplantation rénale, ladite méthode comprenant les étapes suivantes :

   a) mesurer la charge virale de BK-v dans un échantillon biologique dudit patient,
   b) mesurer l'intensité normalisée de la réponse des lymphocytes T CD4$^+$ ou CD8$^+$ mémoires spécifiques du virus BK-v dans un échantillon biologique dudit patient, ladite normalisation étant effectuée par rapport à la charge virale BK-v mesurée à l'étape a), et

c) déterminer les différences d'allèles HLA entre le donneur de rein et ledit patient, de préférence le nombre d'incompatibilités entre les HLA I et HLA II du donneur de rein et dudit patient,

d) évaluer le risque de développer une néphropathie à BK virus (BK-v) chez un patient ayant subi une transplantation rénale en combinant les paramètres déterminés aux étapes a), b) et c).

2. Méthode selon la revendication 1, dans laquelle les trois paramètres a), b) et c) sont ensuite compilés pour créer un index de stratification du risque que le patient développe une néphropathie à BK virus.

3. Méthode selon la revendication 1 ou 2, dans laquelle la charge virale de BK-v est mesurée à l'étape a) par PCR quantitative à partir d'un échantillon de sang total ou de plasma dudit patient.

4. Méthode selon la revendication 1, 2 ou 3, dans laquelle la réponse des lymphocytes T CD4$^+$ ou CD8$^+$ mémoires spécifiques de BK-v mesurée à l'étape b) est déterminée en mettant en contact des cellules mononuclées du sang périphérique dudit patient avec des peptides du virus BK-v, et en évaluant la prolifération des lymphocytes T présents dans lesdites cellules mononuclées après 4 à 7 jours de culture, typiquement après 5 jours de culture.

5. Méthode selon la revendication 4, dans laquelle ladite prolifération est mesurée en déterminant la proportion de lymphocytes T CD4$^+$ ayant dilué un marqueur de prolifération après 4 à 7 jours de culture en présence desdits peptides, typiquement après 5 jours de culture.

6. Méthode selon la revendication 4, dans laquelle ladite prolifération est mesurée en déterminant la proportion de lymphocytes T CD8$^+$ ayant dilué un marqueur de prolifération après 4 à 7 jours de culture en présence desdits peptides, typiquement après 5 jours de culture.

7. Méthode selon la revendication 5 ou 6, dans laquelle ledit marqueur de prolifération est le carboxyfluorescein succinimidyl ester (CFSE) ou le Ki67.

8. Méthode selon l'une des revendications 1 à 7, dans laquelle on conclut à un risque élevé de développer une néphropathie à BK virus (BK-v) lorsque :

a) ramenée à une charge virale de 10$^3$ copies de virus BK-v par mL de sang,

b) l'intensité normalisée de la réponse des lymphocytes T CD4$^+$ ou CD8$^+$ mémoires spécifiques de BK-v est inférieure ou égale à 100 unités de mesure d'intensité, et

c) le nombre d'incompatibilité entre ledit patient et le donneur du rein greffé est inférieur ou égal à 5.

9. Méthode selon l'une des revendications 1 à 8, dans laquelle on conclut à un risque faible de développer une néphropathie à BK virus (BK-v) lorsque :

a) ramenée à une charge virale de 10$^3$ copies de virus BK-v par ml de sang,

b) l'intensité normalisée de la réponse des lymphocytes T CD4$^+$ ou CD8$^+$ mémoires spécifiques de BK-v est strictement supérieures à 100 unités de mesure d'intensité, et

c) le nombre d'incompatibilité entre ledit patient et le donneur de greffe est strictement supérieur à 5.

10. Méthode *in vitro* d'évaluation de la réponse d'un patient greffé rénal à une réplication sanguine du BK-v après modification du traitement immunosuppresseur, **caractérisée en ce que** la méthode définie aux revendications 1 à 9 est répétée à intervalle de temps régulier après la transplantation.

11. Méthode selon la revendication 10, **caractérisée en ce que** la méthode définie aux revendications 1 à 9 est répétée avant et après chaque modification de traitement.

12. Méthode selon la revendication 10, **caractérisée en ce que** la méthode définie aux revendications 1 à 9 est répétée tous les 3 à 6 mois environ.

13. Utilisation d'un kit contenant :

a) des réactifs permettant la réalisation d'une PCR quantitative du virus BK-v, et

b) des séquences peptidiques du BK-v ou des séquences peptidiques polyclonales de stimulation lymphocytaire,

pour mettre en œuvre la méthode des revendications 1 à 12.

14. Utilisation selon la revendication 13, dans laquelle les réactifs b) contiennent également des réactifs de marquage lymphocytaire.

15. Utilisation selon la revendication 13, dans laquelle le kit contient :

a) des amorces spécifiques permettant d'amplifier des portions du génome du BK-v, et
b) des séquences peptidiques du BK-v, de préférence des mélanges de peptides chevauchants, tels que Peptivator®BKV VP1 ou Peptivator®BKV LT et/ou des séquences peptidiques polyclonales de stimulation lymphocytaire,
et, optionnellement,
c) les réactifs nécessaires à la réalisation d'une PCR quantitative avec extraction d'ADN et/ou
d) des réactifs de marquage lymphocytaire.

**Patentansprüche**

1. Verfahren zur Bewertung des Risikos der Entwicklung einer BK-Virus (BK-v)-Nephropathie bei einem Patienten, der sich einer Nierentransplantation unterzogen hat, wobei das Verfahren die folgenden Schritte umfasst:

a) Messen der BK-v-Viruslast in einer biologischen Probe des Patienten,
b) Messen der normalisierten Intensität der Reaktion der BK-v-spezifischen Gedächtnis-T-Lymphozyten CD4$^+$ oder CD8$^+$ in einer biologischen Probe des Patienten, wobei die Normalisierung in Bezug auf die in Schritt a) gemessene BK-v-Viruslast erfolgt, und
c) Bestimmen der Unterschiede der HLA-Allele zwischen dem Nierenspender und dem Patienten, vorzugsweise der Anzahl der Inkompatibilität(en) zwischen HLA I und HLA II des Nierenspenders und des Patienten,
d) Bewerten des Risikos der Entwicklung einer BK-Virus (BK-v)-Nephropathie bei einem Patienten, der sich einer Nierentransplantation unterzogen hat, durch Kombination der in den Schritten a), b) und c) bestimmten Parameter.

2. Verfahren nach Anspruch 1, wobei die drei Parameter a), b) und c) anschließend zusammengestellt werden, um einen Index zur Stratifizierung des Risikos zu erstellen, dass der Patient eine BK-Virus-Nephropathie entwickelt.

3. Verfahren nach Anspruch 1 oder 2, wobei die BK-v-Viruslast in Schritt a) durch quantitative PCR ausgehend von einer Vollblut- oder Plasmaprobe des Patienten gemessen wird.

4. Verfahren nach Anspruch 1, 2 oder 3, wobei die in Schritt b) gemessene Reaktion der BK-v-spezifischen Gedächtnis-T-Lymphozyten CD4$^+$ oder CD8$^+$ bestimmt wird, indem mononukleäre Zellen aus dem peripheren Blut des Patienten mit Peptiden des BK-v-Virus in Kontakt gebracht werden und die Proliferation der in den mononukleären Zellen vorhandenen T-Lymphozyten nach 4 bis 7 Tagen Kultivierung, typischerweise nach 5 Tagen Kultivierung, bewertet wird.

5. Verfahren nach Anspruch 4, wobei die Proliferation gemessen wird, indem der Anteil an T-Lymphozyten CD4$^+$ bestimmt wird, die einen Proliferationsmarker nach 4 bis 7 Tagen Kultivierung in Gegenwart der Peptide, typischerweise nach 5 Tagen Kultivierung, verdünnt haben.

6. Verfahren nach Anspruch 4, wobei die Proliferation gemessen wird, indem der Anteil an T-Lymphozyten CD8$^+$ bestimmt wird, die einen Proliferationsmarker nach 4 bis 7 Tagen Kultivierung in Gegenwart der Peptide, typischerweise nach 5 Tagen Kultivierung, verdünnt haben.

7. Verfahren nach Anspruch 5 oder 6, wobei der Proliferationsmarker Carboxyfluoresceinsuccinimidylester (CFSE) oder Ki67 ist.

8. Verfahren nach einem der Ansprüche 1 bis 7, wobei auf ein erhöhtes Risiko für die Entwicklung einer BK-Virus (BK-v)-Nephropathie geschlossen wird, wenn:

a) geführt auf eine Viruslast von $10^3$ BK-v-Viruskopien pro ml Blut,

b) die normalisierte Intensität der Reaktion der BK-v-spezifischen Gedächtnis-T-Lymphozyten CD4$^+$ oder CD8$^+$ kleiner oder gleich 100 Intensitätsmesseinheiten ist und
c) die Inkompatibilitätszahl zwischen dem Patienten und dem Spender der transplantierten Niere kleiner oder gleich 5 ist.

9. Verfahren nach einem der Ansprüche 1 bis 8, wobei auf ein geringes Risiko für die Entwicklung einer BK-Virus (BK-v)-Nephropathie geschlossen wird, wenn:

a) geführt auf eine Viruslast von 10$^3$ BK-v-Viruskopien pro ml Blut,
b) die normalisierte Intensität der Reaktion der BK-v-spezifischen Gedächtnis-T-Lymphozyten CD4$^+$ oder CD8$^+$ strikt größer als 100 Intensitätsmesseinheiten ist und
c) die Inkompatibilitätszahl zwischen dem Patienten und dem Spender der transplantierten Niere größer als 5 ist.

10. In-vitro-Methode zur Bewertung der Reaktion eines nierentransplantierten Patienten auf eine Blutreplikation von BK-v nach Änderung der immunsuppressiven Behandlung, **dadurch gekennzeichnet, dass** das in den Ansprüchen 1 bis 9 definierte Verfahren in regelmäßigen Zeitabständen nach der Transplantation wiederholt wird.

11. Verfahren nach Anspruch 10, **dadurch gekennzeichnet, dass** das in den Ansprüchen 1 bis 9 definierte Verfahren vor und nach jeder Änderung der Behandlung wiederholt wird.

12. Verfahren nach Anspruch 10, **dadurch gekennzeichnet, dass** das in den Ansprüchen 1 bis 9 definierte Verfahren etwa alle 3 bis 6 Monate wiederholt wird.

13. Verwendung eines Kits, das enthält:

a) Reagenzien zur Durchführung einer quantitativen PCR des BK-v-Virus und
b) BK-v-Peptidsequenzen oder polyklonale Lymphozytenstimulations-Peptidsequenzen

zur Durchführung des Verfahrens der Ansprüche 1 bis 12.

14. Verwendung nach Anspruch 13, wobei die Reagenzien b) ebenfalls Reagenzien zur Lymphozytenmarkierung enthalten.

15. Verwendung nach Anspruch 13, wobei das Kit enthält:

a) spezifische Primer, die es ermöglichen, Teile des BK-v-Genoms zu amplifizieren, und
b) BK-v-Peptidsequenzen, vorzugsweise Gemische überlappender Peptide, wie Peptivator®BKV VP1 oder Peptivator®BKV LT und/oder polyklonale lymphozytenstimulierende Peptidsequenzen, und optional
c) die zur Durchführung einer quantitativen PCR mit DNA-Extraktion erforderlichen Reagenzien und/oder
d) Reagenzien zur Lymphozytenmarkierung.

## Claims

1. Method for assessing the risk of developing BK virus (BK-v) nephropathy in a renal transplant patient, said method comprising the following steps

a) measuring the viral load of BK-v in a biological sample from said patient,
b) measuring the normalized intensity of the BK-v virus-specific memory CD4$^+$ or CD8$^+$ T lymphocyte response in a biological sample from said patient, said normalization being performed with respect to the BK-v viral load measured in step a), and
c) determining the differences in HLA alleles between the kidney donor and said patient, preferably the number incompatibilities between the HLA I and HLA II of the kidney donor and said patient,
d) assessing the risk of developing BK virus (BK-v) nephropathy in a kidney transplant patient by combining the parameters determined in steps a), b) and c).

2. Method according to claim 1, in which the three parameters a), b) and c) are then compiled to create a stratification index of the patient's risk of developing BK virus nephropathy.

3. Method according to claim 1 or 2, wherein the viral load of BK-v is measured in step a) by quantitative PCR from a whole blood or plasma sample of said patient.

4. Method according to claim 1, 2 or 3, in which the response of the BK-v-specific CD4$^+$ or CD8$^+$ memory T lymphocytes measured in step b) is determined by contacting peripheral blood mononuclear cells from said patient with BK-v virus peptides, and by assessing the proliferation of T lymphocytes present in said mononuclear cells after 4 to 7 days of culture, typically after 5 days of culture.

5. Method according to claim 4, wherein said proliferation is measured in determining the proportion of CD4$^+$ T lymphocytes having diluted a proliferation marker after 4 to 7 days of culture in the presence of said peptides, typically after 5 days of culture.

6. Method according to claim 4, wherein said proliferation is measured by determining the proportion of CD8$^+$ T lymphocytes having diluted a proliferation marker after 4 to 7 days of culture in the presence of said peptides, typically after 5 days of culture.

7. Method according to claim 5 or 6, wherein said proliferation marker is the carboxyfluorescein succinimidyl ester (CFSE) or Ki67.

8. Method according to one of claims 1 to 7, in which a high risk of developing BK virus (BK-v) nephropathy is concluded when:

   a) in relation with a viral load of $10^3$ BK-v copies per mL of blood,
   b) the normalized intensity of the response of BK-v-specific memory CD4$^+$ or CD8$^+$ T lymphocytes is less than or equal to 100 intensity measurement units, and
   c) the number incompatibilities between the said patient and the donor of the transplanted kidney is less than or equal to 5.

9. Method according to one of claims 1 to 8, in which a low risk of developing BK virus (BK-v) nephropathy is concluded when:

   a) in relation with a viral load of $10^3$ BK-v copies per ml of blood,
   b) the normalized intensity of the response of BK-v-specific memory CD4$^+$ or CD8$^+$ T lymphocytes is strictly greater than 100 intensity measurement units, and
   c) the number of incompatibilities between the said patient and the transplant donor is strictly greater than 5.

10. An *in vitro* method for assessing the response of a renal transplant patient to BK-v blood replication after modification of immunosuppressive treatment, **characterized in that** the method defined in claims 1 to 9 is repeated at regular time intervals after transplantation.

11. Method according to claim 10, **characterized in that** the method defined in claims 1 to 9 is repeated before and after each treatment modification.

12. Method according to claim 10, **characterized in that** the method defined in claims 1 to 9 is repeated approximately every 3 to 6 months.

13. Use of a kit containing:

   a) reagents for conducting quantitative PCR of BK-v virus, and
   b) BK-v peptide sequences or lymphocyte-stimulating polyclonal peptide sequences, to implement the method of claims 1 to 12.

14. Use according to claim 13, in which the b) reagents also contain lymphocyte labeling reagents.

15. Use according to claim 13, wherein the kit contains:

a) specific primers for amplifying portions of the BK-v genome, and

b) BK-v peptide sequences, preferably mixtures of overlapping peptides such as Peptivator BKV VPI or Peptivator BKV LT and/or lymphocyte-stimulating polyclonal peptide sequences,

and, optionally,

c) reagents necessary for conducting quantitative PCR with DNA extraction and/or

d) lymphocyte labeling reagents.

12 - 24 mois

6 - 12 mois

0 - 6 mois

Dysfonction chronique et
perte du greffon rénal
> 50% en cas de Nx BK-v

Nx BK-v
(Nx BK-v avec réactivations sanguine et urinaire du BK-v associées)
≈ 10 % des patients transplantés rénaux

Virémie à BK-v isolée
(Réactivations sanguine et urinaire du BK-v, sans Nx BK-v identifiée)
≈ 25 % des patients transplantés rénaux

Virurie à BK-v
(Réactivation urinaire stable et isolée du BK-v. Pas de réactivation sanguine, ni de Nx BK-v)
≈ 40 % des patients transplantés rénaux

**Transplantation rénale**

Séroprévalence du BK-v > 80% dans la population générale
(Réactivations urinaires infra-cliniques intermitentes possibles. Pas de réactivation sanguine, ni Nx BK-v)

**Figure 1**

**Figure 2**

## a - Evaluation de la fonction rénale selon les formes de réactivation du virus BK-v

## b - Survie du greffon rénal selon les formes de réactivation du virus BK-v

## Approche actuelle de la Néphropathie à BK-virus (Nx BK-v)

* : cas des Nx BK-v non prouvées histologiquement mais avec PCR BK-virus sanguine $\geq 10^4$ copies/ml pendant plus de 4 semaines

**Figure 3**

**Figure 4**

Combinaison de trois paramètres :

**Figure 5**

## Charge virale du virus BK-v
## dans les différents groupes de patients de la cohorte

a - Copies/mL
dans le sang

Nb valeurs/patient:  13 [10-24]    14[10-18]    8 [6-18]    11 [5-19]

b - Copies/mL
dans l'urine

Nb valeurs/patient:  9 [6-16]    11 [6-15]    9 [6-13]    7 [4-13]

**Figure 6**

1/ Réponse lymphocytaire T mémoire anti-BK-v : stimulation spécifique par les peptides du virus BK-v

## a - Sécrétions cytokiniques

Figure 6 (suite)

b - Prolifération lymphocytaire

**Figure 6 (suite)**

2/ Réponse lymphocytaire T mémoire anti-virale globale : stimulation par un pool de peptides anti-viraux couvrant les virus Cytomégalovirus, Epstein-Barr et Influenza

a – Sécrétions cytokiniques

**Figure 6 (suite)**

b – Prolifération

Figure 7

Intensité de la réponse proliférative des LTm anti-BK-v
en fonction de la charge virale  BK-v (copies/mL de sang)

**Figure 8**

5 jours

a - Prélèvement sanguin

b - PBMC

c - Mise en culture

d - Identification des lymphocytes T (LT)

e - Lymphocytes T (LT) CFSE$^{low}$ (%)

Virémie BK-v isolée

Nx BK-v

Stimulation polyclonale (mitogène)

LT CD8 = 50

LT CD8 = 50

Stimulation spécifique par le BK-v

LT CD8 = 3,20

LT CD8 = 0,24

Pas de stimulation

LT CD8 = 0,08

LT CD8 = 0,12

| Evaluation de l'intensité normalisée de la réponse LTm anti-BK-v | Virémie BK-v | Nx BK-v 1 | Nx BK-v 2 |
|---|---|---|---|
| a - Proportion des LTm anti-BK-v CD8 CFSE$^{low}$ (%) | 3,12 | 0,12 | 0,16 |
| b - Proportion des LTm anti-BK-v CD8 CFSE$^{low}$ exprimée pour $1.10^6$ lymphocytes T CD8 totaux (%*$10^4$) | 31 200 | 1 200 | 1 600 |
| c - Charge virale BK-v (copies/ml) | $2,0.10^4$ | $2,1.10^4$ | $4,3.10^5$ |
| d – **Intensité normalisée de la réponse LTm anti-BK-v** <br> <u>Unité de mesure d'intensité normalisée</u> <br> Proportion des LTm anti-BK-v CD8 CFSE$^{low}$ (%) exprimée pour $1.10^6$ lymphocytes T CD8 totaux (%*$10^4$) et normalisée pour $1.10^3$ copies de BK-v par mL de sang | 1 560 | 57 | 4 |

Figure 9

## Intensité de la réponse LTm anti-BK-v normalisée
## pour $10^3$ copies/mL de BK-v (nombre de lymphocytes T CFSE$^{low}$)

Figure 10

| LTm BK-v fonctionnels (unité de mesure d'intensité normalisée) | CD4 | CD8 |
|---|---|---|
| Virémie à BK-v (n=11) | 810 [1.6 - 8897] | 448.9 [0.1 - 31281] |
| Nx BK-v (n=16) | 2.1 [0.1 - 21.5] | 0.1 [0.1 - 5.9] |
| p | 0.0016 | 0.0001 |

Figure 11

Figure 12

Nombre total d'incompatibilités des molécules HLA

≤ 5 → Intensité de la réponse LTm anti-BK-v normalisée pour $10^3$ copies de BK-v/ml de sang
- ≤ 100 → Risque de Nx BK-v : 100 %
- >100 → 67 %

>5 → Intensité de la réponse LTm anti-BK-v normalisée pour $10^3$ copies de BK-v/ml de sang
- ≤ 100 → 63 %
- >100 → 0 %

Figure 13

## Risque de Nx BK-v en fonction :
i. de l'intensité de la réponse LTm anti-BK-v,
ii. normalisée à la charge virale BK-v dans le sang et
iii. du nombre d'incompatiblités HLA

**Figure 14**

## Indications de la méthode de l'invention

| 1 | 2 | 3 |
|---|---|---|
| Virémie BK-v | Intensification du traitement immunosuppresseur | Dégradation inexpliquée de la fonction du greffon |

Répétition de la méthode tous les 2 à 3 mois après modification du traitement immunosuppresseur

### Objectifs :

Aide au diagnostic et stratification du risque de survenue de Nx BK-v

Stratification du risque de survenue ultérieure de Nx BK-v
➡ Aide à l'adaptation du traitement immunosuppresseur

Aide au diagnostic de Nx BK-v

## Approche proposée pour évaluer le risque de Néphropathie à BK-virus (Nx BK-v)

**Figure 15**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **HIRSCH et al.** *Clinical Microbiology and Infection*, 2014
- Kidney Disease: Improving Global Outcomes (KDIGO) Transplant Work Group. KDIGO clinical practice guideline for the care of kidney transplant recipients. *Am J Transplant.*, 2009, vol. 9, 1-155
- *Rapport Annuel REIN (Réseau Epidémiologie et Information en Néphrologie*, 2015, https://www. agence-biomedecine.fr
- **MAILLART E ; TAOUFIK Y ; GASNAULT J ; STANKOFF B.** Leucoencéphalopathie multifocale progressive. *EMC Neurol.*, 2017
- **DALIANIS T ; HIRSCH HH**. Human polyomaviruses in disease and cancer. *Virology*, 2013, vol. 437, 63-72
- **HIRSCH H ; VINCENTI F ; FRIMAN S ; TUNCER M ; CITTERIO F ; WIECEK A et al.** Polyomavirus BK replication in de novo kidney transplant patients receiving tacrolimus or cyclosporine: a prospective, randomized, multicenter study. *Am J Transplant.*, 2013, vol. 13, 136-45
- **HIRSCH H ; KNOWLES W ; DICKENMANN M ; PASSWEG J ; KLIMKAIT T ; MIHATSCH MJ et al.** Prospective study of polyomavirus type BK replication and nephropathy in renal-transplant recipients. *N Engl J Med.*, 2002, vol. 347, 488-96
- **HIRSCH H ; BABEL N ; COMOLI P ; FRIMAN V ; GINEVRI F ; JARDINE A et al.** European perspective on human polyomavirus infection, replication and disease in solid organ transplantation. *Clin Microbiol Infect.*, 2014, vol. 20, 74-88
- **DEKEYSER M ; FRANÇOIS H ; BEAUDREUIL S ; DURRBACH A.** Polyomavirus-Specific Cellular Immunity: From BK-Virus-Specific Cellular Immunity to BK-Virus-Associated Nephropathy?. *Front Immunol.*, 2015, vol. 6, 307
- **SHARMA R. ; TZETZO S. ; PATEL S. ; ZACHARIAH MAREENA, SHARMA S. ; MELENDY T.** BK virus in Kidney Transplant: current Concepts, Recent Advances and Future Directions. *xperimental and Clinical Transplantation*, 2016, vol. 4, 377-384
- **LIMAYE AP ; JEROME KR ; KUHR CS ; FERRENBERG J ; HUANG ML ; DAVIS CL ; COREY L ; MARSH CL.** Quantitation of BK virus load in serum for the diagnosis of BK virus associated nephropathy in renal transplant recipients. *J. Infect. Dis.*, 2001

- **AWADALLA Y. ; RANDHAWA P. ; RUPPERT K. ; ZEEVI A. ; DUQUESNOY RJ**. HLA Mismatching Increases the Risk of BK Virus Nephropathy in Renal Transplant Recipients. *Am. J. Transplant*, 2004
- **SESTER M ; LEBOEUF C ; SCHMIDT T ; HIRSCH H**. The 'ABC' of Virus-Specific T Cell Immunity in Solid Organ Transplantation. *Am. J. Transplant.*, 2016, vol. 16, 1697-1706
- **RESTIFO, NICHOLAS P ; LUCA GATTINONI**. Lineage Relationship of Effector and Memory T Cells. *Current Opinion in Immunology*, 2013, vol. 25 (5), 556-63
- **EGLI, A. ; HUMAR, A. ; KUMAR, D.** State-of-the-art monitoring of cytomegalovirus-specific cell-mediated immunity after organ transplant: a primer for the clinician. *Clin. Infect. Dis.*, 2012, vol. 55, 1678-1689
- **DRACHENBERG, CINTHIA B. ; JOHN C. PAPADIMITRIOU ; DEAN MANN ; HANS H. HIRSCH ; RAVINDER WALI ; EMILIO RAMOS**. Negative Impact of Human Leukocyte Antigen Matching in the Outcome of Polyomavirus Nephropathy. *Transplantation*, 2005, vol. 80 (2), 276-78
- **HÄSSIG, A. ; M. ROOS ; A. ETTER ; W. BOSSART ; N. MÜLLER ; M. SCHIESSER ; R. P. WÜTHRICH ; T. FEHR**. Association of BK Viremia with Human Leukocyte Antigen Mismatches and Acute Rejection, but Not with Type of Calcineurin Inhibitor. *Transplant Infectious Disease*, 2014, vol. 16 (1), 44-54
- **HELANTERÄ, ILKKA ; KAIJA SALMELA ; LAURI KYLLÖNEN ; ANNE RÄISÄNEN-SOKOLOWSKI ; EEVA AUVINEN ; LAURA MANNONEN ; PETRI KOSKINEN ; IRMELI LAUTENSCHLAGER**. BK Virus Viremia in a Well-HLA-Matched Kidney Transplant Population Mainly on Low-Dose Cyclosporine-Based Immunosuppression. *Clinical Transplantation*, 2012, vol. 26 (6), E596-601
- **THANGARAJU, SOBHANA ; JAGBIR GILL ; ALLISSA WRIGHT ; JIANGHU DONG ; CAREN ROSE ; JOHN GILL**. Risk Factors for BK Polyoma Virus Treatment and Association of Treatment With Kidney Transplant Failure: Insights From a Paired Kidney Analysis. *Transplantation*, 2016, vol. 100 (4), 854-61
- **RAMOS E ; DRACHENBERG CB ; PAPADIMITRIOU JC et al.** Clinical course of polyoma virus nephropathy in 67 renal transplant patients. *J Am Soc Nephrol*, 2002, vol. 13, 2145

- **BATAL I. et al.** Measurments of Global Cell-Mediated Immunity in Renal Transplant Recipients With BK virus reactivation. *Am. J. Clin. Pathol.*, 2008, vol. 129, 857-591
- **COMOLI P. et al.** Polyomavirus-associated nephropathy : update on BK virus-specific immunity. *Transpl. Infect. Dis.*, 2006, vol. 8, 86-94
- **SCHACHTNER T. et al.** BY virus-Specific Immunity Kinetics: A predictor of Recovery From polyomavirus BK-associated nephropathy. *Am. Journal. Of Transplantation*, 2011, vol. 11, 2443-2452
- **SCHACHTNER T. et al.** The Loss of BKV-specific immunity from Pretransplantation to Posttransplantation identifies Kidney Transplant Recipients at Increased Risk of BKV Replication. *Am. Journal. Of Transplantation*, 2015, vol. 15, 2159-2169
- **JOHNSTON, O. et al.** Treatment of polyomavirus infection in kidney transplant recipients: a systematic review. *Transplantation*, 2010, vol. 89, 1057-1070
- **CHUNG, B. H. et al.** Clinical usefulness of BK virus plasma quantitative PCR to prevent BK virus associated nephropathy. *Transpl. Int.*, 2012, vol. 25, 687-695
- **GODINHO PINTO, G.** ; **POLONI, J.** ; **ROTTA, L., R.** ; **RAZONABLE, R.** ; **PASQUALOTTO, A.** Screening for BK virus nephropathy in kidney transplant recipients: comparison of diagnostic tests. *J. Bras. Nefrol.*, 2016, vol. 38
- **COMOLI, P. et al.** Immunity to Polyomavirus BK Infection: Immune Monitoring to Regulate the Balance between Risk of BKV Nephropathy and Induction of Alloimmunity. *Clin. Dev. Immunol.*, 2013, vol. 2013, 256923
- *Le rapport médical et scientifique du prélèvement et de la greffe en France en 2016*, 2016
- **SULEIMAN B.** ; **SALEH S.** ; **HASSAN W** ; **WANG K.** ; **MAIBAM A.** ; **KARIM A.** ; **HINES A.** ; **MEI X.** ; **EL-HUSSEINI A.** ; **YASEEN M.** HLA Mismatching and cPRA Do Not Affect BK Viremia and Nephropathy Risk in Kidney Transplantation: A Retrospective Analysis. *American Transplant Congress*, 2018
- **LANOT A.** ; **BOUVIER N.** ; **CHATELET V.** ; **DINA J.** ; **BÉCHADE C.** ; **FICHEUX M.** ; **HENRI P.** ; **LOBBE-DEZ T.** ; **HURAULT DE LIGNY B.** Infections à BK virus en transplantation rénale. *Nephrologie & therapeutique*, 2016, vol. 12 (2)